# EUROPEAN PATENT APPLICATION

(11) **EP 3 659 603 A1**
(43) Date of publication of application: **03.06.2020**
(21) Application number: 19206293.3
(22) Date of filing: 08.01.2015
(51) Int. Cl.: A61K 31/4745, A61P 35/00, A61P 13/10, A61P 43/00, A61K 9/00, A61K 47/10, A61K 47/12, A61K 47/40

(54) **PHARMACEUTICAL COMPOSITIONS COMPRISING IMIQUIMOD FOR USE IN THE TREATMENT OF CARCINOMA IN SITU OF THE BLADDER**

(30) Priority: 10.01.2014 WO PCT/EP2014/000045
(62) Divisional of application: 15702368.0
(71) Applicant: Urogen Pharma Ltd., 4365007 Ra`anana (IL)
(72) Inventor: Holldack, Johanna, 6815 Melide (CH); Holden, Stuart, 90067 Los Angeles, CA (US); Pozzi, Raffaella, 21020 Brunello (IT)
(74) Representative: Graf von Stosch Patentanwaltsgesellschaft mbH

(57) **Abstract**

The present invention relates to the field of cancer treatment, in particular to the treatment of carcinoma in situ bladder cancer and the provision of pharmaceutical compositions for use in the treatment thereof. The pharmaceutical compositions of the present invention comprise imiquimod, at least one pharmaceutically acceptable excipient, at least one organic acid and at least one thermo-sensitive agent. The present invention further relates to methods of treatment for carcinoma in situ bladder cancer and methods of administering the inventive pharmaceutical composition.

## Description

### FIELD OF THE INVENTION

The present invention relates to the field of cancer treatment, in particular to the treatment of carcinoma in situ of the bladder and the provision of pharmaceutical compositions for use in the treatment thereof. The present invention further pertains to methods of treatment for carcinoma in situ, as well as methods for administering the inventive pharmaceutical compositions.

### BACKGROUND OF THE INVENTION

Bladder Cancer (BC) is the most common malignancy of the urinary tract and the seventh most common cancer in men and the 17th most common cancer in women. The worldwide age standardised incidence rate is 9 per 100,000 for men and 2 per 100,000 for women (2008 data). In the European Union (EU) age standardised incidence rate is 27 per 100, 000 for men and six per 100,000 for women. Incidence varies between regions and countries; in Europe, the highest age standardised incidence rate has been reported in Spain (41.5 in men and 4.8 in women) and the lowest in Finland (18.1 in men and 4.3 in women) (cf. Guidelines on Non-muscle-invasive Bladder Cancer (TaT1 and CIS); European Association of Urology (EAU), 2013). The world global age standardised mortality rate is 3 for men versus 1 per 100,000 for women. In 2008 bladder cancer was the eighth most common cause of cancer-specific mortality in Europe. In the US early (non-muscle-invasive) bladder cancer (NMIBC) affects approximately 500,000 people.

Carcinoma in situ (CIS, also referred to as Tis) generally describes an early form of cancer that is defined by the absence of invasion of tumor cells into the surrounding tissue, usually before penetration through the basement membrane. Thus, typically in carcinoma in situ, neoplastic cells proliferate in their normal cellular environment. For example, in Bowen's disease (carcinoma in situ of the skin), neoplastic epidermal cells accumulate within the epidermis only, without penetrating into the deeper dermis. For this reason, CIS will usually not form a tumour and the lesion is rather flat (e.g. in the skin, cervix) or follows the existing cyto-architecture of the organ (e.g. in the breast, lung). Carcinoma in situ cancer of the bladder is thus defined as a flat (e.g. non-papillary) high-grade not yet non-muscle-invasive urothelial carcinoma. Accordingly, CIS bladder cancer corresponds already to an actual malignancy. Bladder CIS is thus not a precursor to a malignancy, but an actual malignancy. Molecular biology techniques and clinical experience have demonstrated the highly malignant potential of CIS bladder cancer. Without any treatment, more than 50% of the patients with CIS bladder cancer progress to a muscle-invasive disease.

According to the Tumour, Node, Metastasis (TNM) classification system, papillary tumours confined to the mucosa are generally classified as stage Ta, tumours that have invaded the lamina propria are classified as stage T1. Approximately 75% of patients with bladder cancer present with a disease that is confined to the mucosa (stage Ta), including high-grade tumours that are confined to the submucosa (stage T1). Bladder cancer confined to the mucosa is classified as carcinoma in situ bladder cancer (bladder cancer CIS).

The highly malignant potential of CIS bladder cancer is also recognized and reflected in the cancer staging according to the International Bladder Cancer Group (IBCG): The IBCG proposed the following practical definitions of risk based on review of current practice guidelines for NMIBC: "Low risk": solitary, primary, low-grade (Ta) tumour; "intermediate risk": multiple or recurrent low-grade tumours and "high risk": any T1 and/or high grade and/or CIS (cf. Brausi et al., J. Urol 2011; 186:2185-67). The latter tumours are at high risk of recurrence and progression, with progression to e.g. muscle-invasive disease being the primary concern.

Treatment of CIS bladder cancer generally involves transurethral resection of the bladder tumour (TURB), single immediate postoperative instillation of chemotherapy, intravesical chemotherapy and treatment with bacillus Calmette-Guérin (BCG), which is typically administered intravesically (see, e.g. "Guidelines on non-muscle invasive bladder cancer (TaT1 and CIS)", European Association of Urology, 2013).

According to patient data males are at higher risk of suffering from CIS, in particular males over the age of 60, than compared to women of the same age: Approximately 75% of the patients presenting with intermediate risk bladder cancer are male, and about 80% of the patients presenting with high-risk bladder cancer are male (cf. Witjes et al., BJU Int. 2013 Oct;112(6):742-50). In particular, males diagnosed with intermediate risk cancer (intermediate-risk as defined as multiple or recurrent low-grade tumours (TaG1, TaG2)) which present with 2-7 tumours and who are older than 45 years of age are at greatest risk of developing CIS. This is followed by patients older than 74 years of age presenting with the same pathology.

If concurrent CIS bladder cancer is found in association with muscle-invasive bladder cancer (MIBC), cancer therapy is determined according to the invasive tumour. The detection of CIS with TaT1 tumours increases the risk of recurrence and progression of TaT1 tumours and further treatment is mandatory. Patient data suggests that patients presenting with CIS at the time of first resection and subsequent positive urine cytology, appear to have a higher risk for a recurrence that requires a second TURB.

Generally, CIS bladder cancer cannot be cured by an endoscopic procedure alone, as endoscopic procedures, which are a typical treatment option in papillary cancers, are not sufficiently effective in the treatment of CIS bladder cancer. CIS bladder cancer is often characterized by a diffuse appearance and is thus difficult to visualize, such that surgical treatment and/or removal of the cancer will not be sufficient as a treatment option to remove the diseased cells and/or tissue. Hence, histological diagnosis of CIS bladder cancer must be followed by further treatment, e.g. intravesical BCG instillations or radical cystectomy. No consensus exists about whether conservative therapy (intravesical BCG instillations) or aggressive therapy (radical cystectomy) should be preferred. There has been a lack of randomized trials of instillation therapy and early radical cystectomy as immediate primary treatment. Tumour-specific survival rates after early radical cystectomy for CIS bladder cancer are excellent, but as many as 40-50% of patients may be overtreated.

BCG, which is typically used in the treatment of bladder cancer, is derived from *Mycobacterium bovis,* which was transferred to Albert Calmette and Camille Guerin in 1904. The strain, initially extremely virulent, subsequently underwent more than 230 passages on a bile-potato medium and demonstrated avirulence allowing for its use as a vaccine. Studies by Old and Clarke in the 1950ies (Old et al., Nature 1959; 184:291) were able to show that BCG administration had an inhibitory effect on transplanted tumours in mice.

Patients usually tolerate BCG well, however, serious and potentially fatal toxicity may occur (Lamm, Clin Infect Dis 2000; 31 (Suppl. 3): S86-90). BCG treatment can result in irritative bladder symptoms, which typically begin after the second or third installation and last for 1-2 days. Many patients (as much as up to nearly 60%) therefore discontinue the BCG treatment due to its side effects, which generally include low-grade fever and local (bladder-related) irritative symptoms, such as e.g. mild bladder pain, urine leakage or incontinence.

Severe reactions or infection with BCG results from i.v. absorption of the organism, most commonly from traumatic catheterization. Bleeding arising from a catheter placed under difficulties is thus an absolute contraindication to the instillation of BCG. In patients experiencing continued side-effects due to BCG treatment, logarithmic dose reduction of BCG is considered. Patients suffering from a BCG sepsis or local infections require steroids in addition to gram negative and anti-tubercular antibiotic therapy. Moreover, BCG should also never be used as an immediate postoperative intravesical instiallation, as the risk for bacterial sepsis and possibly death is high. Other contraindications to the use of BCG include for example conditions such as urinary tract infection and immuno-suppression (Sylvester, International Journal of Urology (2011), 113-120).

In addition to the side effects observed in patients, the use of BCG also imposes a health risk on health care workers and physicians handling BCG, as well as patients that are in contact with patients receiving BCG: BCG infections have been reported in health care workers and physicians, primarily from exposures resulting from accidental needle sticks or skin lacerations during the preparation of BCG for administration. Nosocomial infections with BCG have also been reported in patients receiving parenteral drugs. In these cases the drugs were prepared in areas in which BCG was reconstituted and led to a contamination of the drug.

Patients which have undergone treatment with BCG and interferons have a 60%-70% chance of a complete and durable response, if they were never treated with BCG before, or if they failed only one prior induction or relapsed more than a year from induction (Grossmann et al., Rev. Urol. (2008), Vol. 10, No. 4, p. 281 - 289). However, up to 40% of patients eventually fail in intravesical BCG treatment. These patients usually have a poor prognosis with a high risk of progression to invasive disease and death as a result of bladder cancer. Thus, CIS bladder cancer patients that are treated conventionally by e.g. surgery (TURB), established chemotherapy and/or by intravesical BCG administration have a poor prognosis due to the limited success of these treatment options.

Development of new treatment options for the treatment of highly malignant CIS bladder cancer is thus highly desirable. It is therefore an object of the present invention to provide pharmaceutical compositions, which allow for treating patients with CIS bladder cancer with an improved safety and good efficacy profile.

### SUMMARY OF THE INVENTION

The present inventors have identified that pharmaceutical compositions comprising the immune response modifier imiquimod have an unexpectedly good efficacy in treating carcinoma in situ (CIS), the most aggressive form of bladder cancer. The inventors found that the inventive pharmaceutical compositions comprising imiquimod have anti-neoplastic activity in carcinoma in situ bladder cancer.

Accordingly, the present invention provides a pharmaceutical composition, which comprises imiquimod for use in the treatment of carcinoma in situ bladder cancer, e.g. by intravesical administration.

According to one embodiment, the pharmaceutical composition of the present invention comprises imiquimod and at least one pharmaceutically acceptable excipient and optionally a vehicle.

More specifically, the inventive pharmaceutical composition as defined above comprises imiquimod in an amount of about 0.005 % (w/v) to about 2 % (w/v).

According to a further embodiment, the inventive pharmaceutical composition may comprise at least one organic acid to improve the solubility properties of imiquimod in aqueous solution. Accordingly, the inventive pharmaceutical composition may further comprise acetic acid and/or lactic acid, e.g. the inventive pharmaceutical composition may comprise acetic acid, or lactic acid, or both, lactic acid and acetic acid.

According to a more specific embodiment, the inventive pharmaceutical composition for use in the treatment of CIS bladder cancer may further comprise at least one thermo-sensitive agent. More specifically, the inventive pharmaceutical composition may comprise the at least one thermo-sensitive agent, which exhibits a specific "lower critical solution temperature" (LCST) in a range of about 15°C to about 35°C, more preferably in a range of about 15°C to about 30°C, even more preferably in a range of about 15 or 20°C or 25°C to about 30°C, most preferably in a range of about 15 or 20°C to about 25°C. More specifically, the inventive pharmaceutical composition comprises the least one thermo-sensitive agent in an amount of about 0.1 % (w/v) to about 40 % (w/v).

Accordingly, the inventive pharmaceutical composition comprises at least one thermo-sensitive agent selected from a poly(ethylene oxide)-poly(propylene oxide)-poly(ethylene oxide) copolymer (also termed PEO-PPO-PEO or poloxamer) or from chitosan or its derivatives.

According to a more preferred embodiment, the inventive pharmaceutical composition comprises at least one thermo-sensitive agent selected from a poly(ethylene oxide)-poly(propylene oxide)-poly(ethylene oxide) copolymer (also termed PEO-PPO-PEO or poloxamer). Accordingly, the at least one thermo-sensitive agent of the inventive pharmaceutical composition may be Poloxamer 407 and/or Poloxamer 188 or a mixture of Poloxamer 407 and Poloxamer 188.

According to a more preferred embodiment, the inventive pharmaceutical composition further comprises one or more cyclodextrin(s), selected from α-cyclodextrins, β-cyclodextrins, γ-cyclodextrins, δ-cyclodextrins and ε-cyclodextrins, preferably from β-cyclodextrins, including hydroxypropyl-β-cyclodextrin (HP-β-CD).

More specifically, the pharmaceutical composition of the present invention as defined above is an aqueous solution, wherein the pH of the solution is in a range of about pH 4.0 - 5.0, preferably in a range of about pH 4.1 - 4.7.

According to a further aspect the present invention provides for the use of the inventive pharmaceutical composition as defined above for the manufacture of a medicament for the treatment of carcinoma in situ (CIS).

According to another aspect, the present invention provides for a method of treatment of carcinoma in situ, which comprises administering to a person in need thereof a therapeutically active amount of the pharmaceutical composition as defined above. The method may comprise intravesical administration of the inventive pharmaceutical composition as defined above.

More specifically, the present invention provides for the use of the inventive pharmaceutical composition as defined above for intravesical administration. Irrespective of the route of administration, the inventive pharmaceutical composition may be administered, at time intervals of about 2-7 days, preferably at time intervals of 2, 3, 4, 5, or 6 days, more preferred the inventive pharmaceutical composition is administered at time intervals of 3-6 days or at time intervals of 7 days, e.g. once a week. The inventive pharmaceutical composition may be administered for at least 3 weeks, for at least 4 weeks, for at least 5 weeks, for at least 6 weeks, for at least 7 weeks, for at least 8 weeks, preferably for about 4-12 weeks, for about 4-8 weeks, for about 6-12 weeks, even more preferred for about 6- 8 weeks. Accordingly, the inventive pharmaceutical composition as defined above may be administered by a total of at least 3, 4, 5, 6, 7 or at least 8 doses, or by e.g. 4-36 doses.

More specifically, the inventive pharmaceutical composition may be provided such that it is retained in the bladder (intravesical retention time) for at least about 0.5h, 1 h, 1.5h, 2h, 2.5h, 3h, 3.5h or for about 4h, preferably for about 0.5h-2h, or for about 1h. Finally, the inventive pharmaceutical composition may be administered in a volume of about 10-100ml.

Accordingly, the present invention also provides for a method of treatment of CIS bladder cancer, wherein the method comprises administering to a person in need thereof the inventive pharmaceutical composition as defined above.

### DETAILED DESCRIPTION OF THE INVENTION

Although the present invention is described in detail below, it is to be understood that this invention is not limited to the particular methodologies, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

In the following, the elements of the present invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

Throughout this specification and the claims which follow, unless the context requires otherwise, the term "comprise", and variations such as "comprises" and "comprising", will be understood to imply the inclusion of a stated member, integer or step but not the exclusion of any other non-stated member, integer or step. The term "consist of" is a particular embodiment of the term "comprise", wherein any other non-stated member, integer or step is excluded. In the context of the present invention, the term "comprise" encompasses the term "consist of".

The terms "a" and "an" and "the" and similar reference used in the context of describing the invention (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. No language in the specification should be construed as indicating any non-claimed element essential to the practice of the invention.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

Within the context of the pharmaceutical composition of the present invention the term imiquimod refers to the compound 1-Isobutyl-1H-imidazo[4,5-c]quinolin-4-amine of the following structure 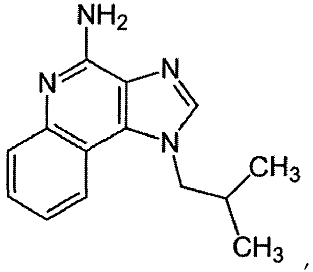 or any pharmaceutically acceptable salt thereof.

Pharmaceutically acceptable salt in the context of the pharmaceutical composition according to the present invention means those salts which retain the biological effectiveness and properties of imiquimod and which are not biologically or otherwise undesirable, formed with inorganic acids such as hydrochloric acid, hydrobromic acid, sulfuric acid, sulfamic acid, nitric acid, phosphoric acid, and the like, and organic acids such as acetic acid, trifluoroacetic acid, adipic acid, ascorbic acid, aspartic acid, benzenesulfonic acid, benzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, hexanoic acid, formic acid, fumaric acid, 2-hydroxyethane- sulfonic acid (isethionic acid), lactic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, pivalic acid, propionic acid, pyruvic acid, salicylic acid, stearic acid, succinic acid, sulfanilic acid, tartaric acid, p-toluenesulfonic acid, undecanoic acid.

It has been surprisingly found that the poorly treatable CIS bladder cancer may be successfully treated by administration of imiquimod, preferably by its intravesical instillation. Accordingly, everything described herein regarding a pharmaceutical composition, in particular a pharmaceutical composition comprising imiquimod, also applies to imiquimod (itself). For example, this applies to the CIS bladder cancer patients to be treated, in particular the preferred groups of patients to be treated, e.g. patients having concurrent CIS bladder cancer, as well as to further embodiments of the pharmaceutical composition, the uses of a pharmaceutical composition according to the present invention and the method of treatment of CIS of the bladder comprising administering a pharmaceutical composition according to the present invention.

The term "bladder cancer" refers to any of several types of (malignant or non-malignant) neoplastic diseases of the bladder arising from the urothelium. The term "carcinoma in situ" (CIS) to be treated according to the present invention preferably refers to flat (e.g. non-papillary), high-grade non-muscle-invasive bladder tumours confined to the mucosa.

Carcinoma in situ can present as a velvet-like reddish area that is indistinguishable from inflammation. In many instances it may not be visible at all. However, CIS bladder cancer patients often show hematoria as an early symptom. Also, CIS patients usually complain of pain symptoms. In order to diagnose CIS bladder cancer, in particular in order to differentiate CIS bladder cancer from other forms of non-muscle-invasive bladder cancer, imaging techniques, in particular ultra-sonography and intravenous urography as well as CT urography, are usually inconclusive and do not have any role in the diagnosis of CIS. Whenever abnormal areas of the urothelium are seen, it is preferred to take biopsies, e.g. biopsies with a resection loop of the abnormal area. The diagnosis of CIS is preferably made by cytoscopy, urine cytology and histological evaluation of bladder biopsies. Cytology is particularly sensitive in CIS detection, as most patients exhibit loss of cohesion of cells in the epithelial lining of the CIS bladder area. Accordingly, a larger number of cells float in the urine. Alternatively or additionally, photodynamic diagnosis by fluorescent cystoscopy may be useful for the detection of CIS, in particular for patients with positive cytology.

Typically, CIS bladder cancer patients do not exhibit T1 stage tumours invading the lamina propria. Under certain circumstances patients may also partially exhibit isolated cancer cells, which are not confined to mucosa, but may be located in the submucosal area. In this transitional stage no evidence of T1 stage tumours may be detectable by conventional methods, such as e.g. histology.

CIS bladder cancer patients may be any female or male of any age. CIS bladder cancer is typically more frequently observed in men than in women with male and female CIS bladder cancer patients equally treated by imiquimod, the patients to be treated are typically males of any ethnicity, preferably males of at least 40 years of age, more preferably males of at least 50 years of age, most preferred males at least 60 years of age, e.g. males in the age of 60-80 years, in the age of 60-70 years. Accordingly, the CIS bladder cancer patients to be treated according to the invention are preferably males in the age of 40-80 years, in the age of 40-70 years, in the age of 50-70 years, in the age of 40-60 years, in the age of 60-80 years, in the age of 45-70 years, in the age of 50-75 years, in the age of 55-80 years, in the age of 65-80 years, more preferably males in the age of 50-75 years, e.g. males in the age of 55-75 years, in the age of 60-75 years, in the age of 65-75 years.

The group of male or female patients that are diagnosed with high-risk tumours, may exhibit at least one, e.g. from 1 to up to 7 tumour sites are treated by imiquimod according to the invention. Preferably the patients to be treated may typically be males older than 60 years of age. Thus, typically the patient group exhibiting with high-risk bladder cancer diagnosed with CIS are male and older than 60 years of age and may e.g. exhibit at least 2 CIS tumour sites.

Patients to be treated may show CIS bladder cancer foci in the upper urinary tract, prostatic ducts and/or the urethra. A typical patient group shows multi-focal cancer occurrence in any of the above body parts.

Carcinoma in situ patients to be treated may be selected from any of the subgroups defined in the following: Patients with primary CIS bladder cancer exhibiting isolated CIS with no previous or concurrent exophytic tumours, in particular with neither previous nor concurrent papillary urothelial tumours , secondary CIS bladder cancer patients showing a history with a previous tumour, in particular with a previous papillary urothelial tumour, or concurrent CIS bladder cancer patients showing CIS in the presence of exophytic tumours, in particular in the presence of a papillary urothelial tumour.

It is thus preferred that the group of male or female, preferably male, CIS bladder patients according to the present invention have a CIS bladder cancer selected from the group consisting of primary CIS bladder cancer, secondary CIS bladder cancer and concurrent CIS bladder cancer. In other words, preferably the carcinoma in situ (CIS) of the bladder is selected from the group consisting of primary carcinoma in situ (CIS) of the bladder, secondary carcinoma in situ (CIS) of the bladder and concurrent carcinoma in situ (CIS) of the bladder.

Thereby, CIS bladder patients having concurrent CIS bladder cancer are particularly preferred according to the present invention. In other words, it is particularly preferred that the carcinoma in situ (CIS) of the bladder is concurrent carcinoma in situ (CIS) of the bladder.

Further, the group of male or female patients, typically male, to be treated according to the invention includes as a preferred specific sub-group patients which previously received intravesical BCG treatment and in which CIS bladder cancer still persists. That sub-group may contribute to about 40% of the patients with CIS bladder cancer.

In general, CIS bladder cancer patients to be treated according to the present invention may have previously, overlapping or concomitantly received/receive a treatment with BCG. CIS bladder patients treated with BCG may be treated according to the present invention, regardless whether the patients responded to the BCG treatment or not. In other words, BCG-responders and BCG-non-responders may be treated according to the present invention. Preferably, however, such CIS bladder cancer patients are treated according to the present invention, which are BCG-non-responders, i.e. which received/receive a BCG treatment, but did not respond to it.

The present inventors surprisingly found that the inventive pharmaceutical composition comprising imiquimod was particularly effective in treating CIS bladder cancer, e.g. in patients presenting with a proven, recurrent primary, secondary or concomitant CIS bladder cancer, which was defined either CIS bladder cancer alone or Ta stage or T1 stage with CIS, preferably provided that the Ta T1 stage tumours were fully resected prior to treatment.

Accordingly, the present invention provides a pharmaceutical composition for use in the treatment of carcinoma in situ of the bladder.

According to one aspect the inventive pharmaceutical composition may comprise at least one pharmaceutically acceptable excipient and optionally a diluent. Accordingly, the inventive pharmaceutical composition may comprise at least 1, or 2, 3 or 4 or more pharmaceutical excipients and optionally a diluent. The term "pharmaceutically acceptable excipients", as used in the context of the inventive pharmaceutical composition, refers to any physiologically inert, pharmacologically inactive material known to one skilled in the art, which is compatible with the physical and chemical characteristics of the pharmaceutical composition according to the invention and which does not interfere with its intended use, such as e.g. intravesical instillation. Pharmaceutically acceptable excipients include, but are not limited to, polymers, resins, plasticizers, fillers, binders, lubricants, glidants, solvents, co-solvents, buffer systems, surfactants, preservatives, sweetening agents, flavoring agents, pharmaceutical grade dyes or pigments, and viscosity agents.

The pharmaceutical grade days in the inventive pharmaceutical composition may also include dyes which e.g. adhere and/or mark the urothelium which was contacted by the inventive pharmaceutical composition, thereby allowing to identify areas which were contacted with the inventive pharmaceutical composition. These areas may be visualized by e.g. endoscopy, or other imaging techniques suitable for imaging the urothelium. Preferably, the fluorescent dyes are non- toxic dyes and fluoresce when exposed to radiant energy, e.g. light. Dyes which may be used with the inventive pharmaceutical composition may include e.g. dansyl chloride, rhodamine isothiocyanate, Alexa 350, Alexa 430, AMCA, aminoacridine, BODIPY 630/650, BODIPY 650/665, BODIPY-FL, BODIPY-R6G, BODIPY-TMR, BODIPY-TRX, 5-carboxy-4',5'-dichloro-2',7'-dimethoxy fluorescein, 5- carboxy-2',4',5',7'-tetrachlor-ofluorescein, 5-carboxyfluorescein, 5-carboxyrhodamine, 6- carboxyrhodamine, 6-carboxy-tetramethyl amino, Cascade Blue, Cy2, Cy3, Cy5,6-FAM, dansyl chloride, fluorescein, HEX, 6-JOE, NBD (7-nitrobenz-2-oxa-1,3-diazole), Oregon Green 488, Oregon Green 500, Oregon Green 514, Pacific Blue, phthalic acid, terephthalic acid, isophthalic acid, cresyl fast violet, cresyl blue violet, brilliant cresyl blue, para- aminobenzoic acid, erythrosine, phthalocyanines, azomethines, cyanines, xanthines, succinylfluoresceins, rare earth metal cryptates, europium trisbipyridine diamine, a europium cryptate or chelate, diamine, dicyanins, La Jolla blue dye, allopycocyanin, allococyanin B, phycocyanin C, phycocyanin R, thiamine, phycoerythrocyanin, phycoerythrin R, REG, Rhodamine Green, rhodamine isothiocyanate, Rhodamine Red, ROX, TAMRA, TET, TRIT (tetramethyl rhodamine isothiol), Tetramethylrhodamine, and Texas Red.

In the context of the pharmaceutical composition according to the present invention, a pharmaceutically acceptable vehicle typically includes a liquid basis of the inventive pharmaceutical composition, e.g. pyrogen-free water; the free water solution may be combined in any appropriate ratio with a water-miscible, pharmaceutically acceptable organic solvent, e.g. an alcohol (e.g. ethanol or isipropanol); the following may also be used: isotonic saline or buffered (aqueous) solutions, e.g phosphate, citrate, etc. buffered solutions, an aqueous buffered solution, containing e.g. a sodium salt, preferably at least 50 mM of a sodium salt, a calcium salt, preferably at least 0.01 mM of a calcium salt, and/or a potassium salt, preferably at least 3 mM of a potassium salt. According to a preferred embodiment, the sodium, calcium and/or potassium salts may occur in the form of their halogenides, e.g. chlorides, iodides, or bromides, in the form of their hydroxides, carbonates, hydrogen carbonates, or sulfates, etc. Without being limited thereto, examples of sodium salts include e.g. NaCl, Nal, NaBr, Na₂CO₃, NaHCO₃, Na₂SO₄, examples of the optional potassium salts include e.g. KCI, Kl, KBr, K₂CO₃, KHCO₃, K₂SO₄, and examples of calcium salts include e.g. CaCl₂, Cal₂, CaBr2, CaCO₃, CaSO₄, Ca(OH)₂. Furthermore, organic anions of the aforementioned cations may be contained in the composition of the invention. According to a more preferred embodiment, the composition of the invention suitable for injection purposes as defined above, may contain salts selected from sodium chloride (NaCI), calcium chloride (CaCl₂) and optionally potassium chloride (KCI), wherein further anions may be present additional to the chlorides. CaCl₂ can also be replaced by another salt like KCI. The composition of the invention may be hypertonic, isotonic or hypotonic with reference to the specific reference medium, i.e. the composition of the invention may have a higher, identical or lower salt content with reference to the specific reference medium, wherein preferably such concentrations of the afore mentioned salts may be used, which do not lead to damage of cells due to osmosis or other concentration effects. Reference media are e.g. liquids occurring in *"in vivo"* methods, such as blood, lymph, cytosolic liquids, or other body liquids, or e.g. liquids, which may be used as reference media in *"in vitro"* methods, such as common buffers or liquids. Such common buffers or liquids are known to a skilled person.

According to a preferred embodiment the pharmaceutical composition according to the present invention typically comprises imiquimod in an amount of about 0.005 % (w/v) to about 2 % (w/v), in an amount of about 0.01 % (w/v) to about 2 % (w/v), in an amount of about 0.1 % (w/v) to about 1.5 % (w/v), in an amount of about 0.1 % (w/v) to about 1.25 % (w/v), an amount of about 0.1 % (w/v) to about 1 % (w/v), an amount of about 0.2 % (w/v) to about 2 % (w/v), an amount of about 0.2 % (w/v) to about 1.5 % (w/v), an amount of about 0.2 % (w/v) to about 1.25 % (w/v), an amount of about 0.2 % (w/v) to about 1 % (w/v), an amount of about 0.2 % (w/v) to about 0.9 % (w/v), an amount of about 0.3 % (w/v) to about 1 % (w/v), an amount of about 0.3 % (w/v) to about 0.9 % (w/v), an amount of about 0.4 % (w/v) to about 1 % (w/v), an amount of about 0.4 % (w/v) to about 0.8 % (w/v), an amount of about 0.1 % (w/v) to about 0.8 % (w/v), an amount of about 0.2 % (w/v) to about 0.8 % (w/v), an amount of about 0.1 % (w/v) to about 0.5 (w/v), preferably in an amount of about 0.2 % (w/v) to about 0.5 % (w/v). The above values may also apply correspondingly for% (w/w).

According to one embodiment the inventive pharmaceutical composition comprises at least one organic acid. The at least one organic acid for use in the inventive pharmaceutical composition may be a carboxylic acid and/or sulfonic acids, preferably the at least one organic acid is a carboxylic acid selected from lactic acid, acetic acid, formic acid, citric acid, oxalic acid, uric acid, benzoic acid, octanoic acid, icosanoic acid, octadecanoic acid, hexadecanoic acid or dodecanoic acid.

According to a preferred embodiment of the present invention, the inventive pharmaceutical composition as defined above comprises an organic acid selected from acetic acid and/or lactic acid or a mixture thereof in a concentration of about 0.025 M to about 0.200 M, preferably in a concentration of about 0.025 M to about 0.100 M, or in a concentration of about 0.035 M to about 0.1 M, or in a concentration of about 0.045 M to about 0.1 M, or in a concentration of about 0.055 M to about 0.1 M, or in a concentration of about 0.065 M to about 0.1 M, or in a concentration of about 0.075 M to about 0.1 M, or in a concentration of about 0.085 M to about 0.1 M, or in a concentration of about 0.100 M to about 0.200 M, or in a concentration of about 0.100 M to 0.150 M, or in a concentration of about 0.020 M to about 0.200 M, e.g. in a concentration of about 0.025 M to about 0.200 M, of about 0.030 M to about 0.200 M, of about 0.035 M to about 0.200 M, of about 0.040 M to about 0.200 M, of about 0.045 M to about 0.200 M, of about 0.050 M to about 0.200 M, of about 0.055 M to about 0.200 M, of about 0.060 M to about 0.200 M, of about 0.065 M to about 0.200 M, of about 0.070 M to about 0.200 M, of about 0.075 M to about 0.200 M, of about 0.080 M to about 0.200 M, of about 0.085 to 0 about.200 M, of about 0.090 M to about 0.200 M, of about 0.095 M to about 0.200 M, of about 0.100 M to about 0.200 M, of about 0.125 M to about 0.200 M, of about 0.130 M to about 0.200 M, of about 0.135 M to about 0.200 M, of about 0.140 M to about 0.200 M, of about 0.145 M to about 0.200 M, of about 0.0150 M to about 0.200 M, of about 0.155 M to about 0.200 M, of about 0.160 M to about 0.200 M, of about 0.165 M to about 0.200 M, of about 0.170 M to about 0.200 M, of about 0.175 M to about 0.200 M, of about 0.180 M to about 0.200 M, of about 0.185 M to about 0.200 M, of about 0.190 M to about 0.200 M, or about 0.195 M to about 0.200 M, or preferably in a concentration of about 0.030 M to about 0.100 M, e.g. in a concentration of about 0.035 M to about 0.100 M, of about 0.040 M to about 0.100 M, of about 0.045 M to about 0.100 M, of about 0.050 M to about 0.100 M, of about 0.055 M to about 0.100 M, of about 0.060 M to about 0.100 M, of about 0.065 M to about 0.100 M, of about 0.070 M to about 0.100 M, or of about 0.075 M to about 0.100 M, or in a concentration of about 0.080 M to about 0.100 M, of about 0.085 to about 0.100 M, of about 0.090 M to about 0.100 M, of about 0.095 M to about 0.100 M, of about 0.095 M to about 0.200 M.

Accordingly, the pharmaceutical composition of the present invention as defined above may comprise acetic acid and/or lactic acid or a mixture thereof, e.g. the inventive pharmaceutical composition may comprise acetic acid, or lactic acid, or acetic acid and lactic acid in any of the above total concentrations. The inventive pharmaceutical composition may thus comprise, e.g. lactic acid in a concentration in a concentration of about 0.225 % (w/v) to about 1.81 % (w/v), preferably in a concentration of about 0.27 % (w/v) to about 1.81 % (w/v), of about 0.36 % (w/v) to about 1.35 % (w/v), of about 0.45 % (w/v) to about 1.35 % (w/v), of about 0.54 % (w/v) to about 1.13 % (w/v), of about 0.63 % (w/v) to about 0.9 % (w/v), of about 0.72 % (w/v) to about 0.9 % (w/v), more preferably in a concentration of about 0.45 % (w/v) to about 0.9 % (w/v), most preferred in a concentration of about 0.51% (w/v) to about 0.9% (w/v) or any range formed by any of two of these values as defined above. The above values may also apply correspondingly for% (w/w). In the context of the inventive pharmaceutical composition the term "lactic acid" refers to 2-hydroxypropanioc acid and includes both, (R)- and (S)-2-hydroxypropanoic acid as well as any racemic mixture thereof. The at least one organic acid as defined above improves the solubility properties of imiquimod in aqueous solution.

According to a particular embodiment, the inventive pharmaceutical composition may comprise one or more cyclodextrins, which are also designated cycloamyloses. Cyclodextrins may be used to enhance solubility and, advantageously, membrane penetration of imiquimod in the inventive pharmaceutical composition, even though imiquimod may not be sufficiently dissolved by just cyclodextrins. In this context, solubility of the imiquimod is not only enhanced in the final inventive pharmaceutical composition using cyclodextrins, but also in an intermediate (stock) solution formed by imiquimod and an organic acid as defined above, e.g. lactic acid, acetic acid or a mixture thereof.

The use of at least one cyclodextrins in combination with lactic acid and/or acetic acid in the inventive pharmaceutical composition results in a small, but significant increase in the solubility of imiquimod, of at least 10%, more preferably of at least 15% or at least about 18 % compared to the solubility of imiquimod in combination with lactic acid alone.

Accordingly, cyclodextrins may be used in any stage of preparation of the pharmaceutical composition to enhance the solubility of imiquimod. In the context of the present invention, cyclodextrins are preferably understood as members of a family of cyclic oligosaccharides, composed of 5 or more α-D-glucopyranoside units linked between positions 1 and 4, as known for amylose, a fragment of starch. In the context of the present invention, cyclodextrins particularly comprise α-cyclodextrins, which form six membered sugar ring molecules, β-cyclodextrins, which form, seven sugar ring molecules, γ-cyclodextrins, which form eight sugar ring molecules, δ-cyclodextrins and ε-cyclodextrins. Particularly preferred, the inventive pharmaceutical composition comprises α-cyclodextrins, β-cyclodextrins, and/or γ-cyclodextrins, even more preferably, β-cyclodextrins, such as hydroxypropyl-β-cyclodextrin (HP-β-CD).

Accordingly, the inventive pharmaceutical composition as defined above may comprise cyclodextrins as defined above in an amount of about 0.1 % (w/v) to about 30 % (w/v), typically in an amount of about 1 % (w/v) to about 20 % (w/v), preferably in an amount of about 2 % (w/v) to about 20 % (w/v), more preferably in an amount of about 5 % (w/v) to about 20 % (w/v), even more preferably in an amount of about 5 % (w/v) to about 15 % (w/v), and most preferably in an amount of about 5 % (w/v) to about 10 % (w/v), or in an amount of about 2 % (w/v) to about 6 % (w/v), or in an amount of about 0.1 % (w/v) to about 4 % (w/v), or in an amount of about 0.5 % to 5 %, more preferably in an amount of 2 % to 5 % or, alternatively, in an amount of about 4 % (w/v) to about 8 % (w/v), or in an amount of about 6 % (w/v) to about 10 % (w/v), or in an amount of about 8 % (w/v) to about 12 % (w/v), wherein the amounts as defined in % (w/v) may either be understood to be based on the weight of the cyclodextrine with respect to the total volume of the inventive pharmaceutical composition or an intermediate stock solution, when e.g. provided as a liquid or semi-liquid formulation. Alternatively, the above amounts may be defined in % (w/v), wherein the amount as defined in % (w/v) may either be determined on the basis of the weight of the cyclodextrine with respect to the total weight of the inventive pharmaceutical composition or on the basis of an intermediate stock solution.

The inventive pharmaceutical composition as defined above may thus comprise cyclodextrins as defined above in an amount of about 2 % (w/v) to about 6 % (w/v), e.g. of about 2.5 % (w/v) to about 6 % (w/v), of about 3 % (w/v) to about 6 % (w/v), of about 3.5 % (w/v) to about 6 % (w/v), or of about 4.5 % (w/v) to about 6 % (w/v), or of about 2.5 % (w/v) to about 5.5 % (w/v), about 3 % (w/v) to about 5.5 % (w/v), of about 3.5 % (w/v) to about 5.5 % (w/v), of about 4 % (w/v) to about 5.5 % (w/v), of about 4.5 % (w/v) to about 5.5 % (w/v), or of about 5 % (w/v), preferably of about 4 % (w/v) to about 6 % (w/v).

According to a more preferred embodiment, particularly for certain administration forms, such as e.g. intravesical administration, the pharmaceutical composition according to the present invention further comprises at least one thermo-sensitive agent. In the context of the present invention, the term "thermo-sensitive agent" typically refers to a compound, preferably a polymer, which is able to change its state of aggregation or its viscosity at a defined point of transition (cooperative transition) from a liquid or semi-liquid state into a solid or semi solid state, preferably to a solid state. More preferably the term "thermo-sensitive agent" typically refers to a compound, preferably a(n) (organic) polymer, which is able to change its state of aggregation from a liquid or semi-liquid state into a solid or semi solid state (e.g. from a liquid to a gel-like state or to a solid state) at a specific point of transition (also called "lower critical solution temperature" (LCST) or "gel transition temperature"), wherein the specific point of transition is preferably defined by a specific transition temperature in a range of about 15°C to about 35°C, more preferably in a range of about 15°C to about 30°C, even more preferably in a range of about 15 or 20°C to about 30°C, most preferably in a range of about 15 or 20°C to about 25°C. The "lower critical solution temperature" according to the present invention is measured at ambient pressure and depends on the molar-mass distribution of the thermo-sensitive agent.

Preferably, such a thermo-sensitive agent as defined above allows an *in situ* gel formation of the thermo-sensitive agent and any compound or composition formulated therewith, e.g. the inventive pharmaceutical composition at body temperature, whereas the pharmaceutical composition will typically display (semi)liquid properties. In this context, an *in situ* gel formation of the thermo-sensitive agent and any compound or composition formulated therewith typically occurs directly upon or directly subsequent to administration of the inventive pharmaceutical composition to the site of affliction, such as e.g. into the bladder (intravesical administration) of the patient to be treated, i.e. not prior to administration of the inventive pharmaceutical composition.

Such an *in situ* gel formation is in particular advantageous for specific applications, e.g. such as intravesical administration of the inventive pharmaceutical composition as defined herein, for the release of imiquimod over an extended period of time (e.g. 0.5h to about 2 hours, preferably 1 hour) inside the bladder and is therefore particularly suitable for the treatment of carcinoma in situ of the bladder.

One particular advantage of a pharmaceutical composition of the present invention comprising thermo-sensitive agents is the ease of its administration due to selection of a transition point at a temperature range as defined above. More specifically, the selection of a transition point at a temperature range as defined above allows not only for preparation or storage of the inventive pharmaceutical composition in a liquid or semi-liquid aggregate state, e.g. at room temperature. It also allows for administration of the (preferably liquid) inventive pharmaceutical composition by e.g. injection by means of a catheter, since the inventive pharmaceutical composition directly solidifies or undergoes gelation subsequent to administration due to the increased temperature of the surrounding bladder tissue, which is preferably higher than the temperature of the transition point. Thus, gel formation is induced within the bladder.

Administration of the inventive pharmaceutical composition as defined above thereby may be carried out using non-invasive methods (without surgery), such as by means of an injection needle having a cannula of a suitable diameter, an injection tube, endoscopic methods, transurethral catheter and the like.

The gel formation additionally results in increased bioadhesive properties of the inventive pharmaceutical composition of the present invention leading to a prolonged exposure of imiquimod to TLR7-expressing cells and less systemic drug penetration. This way TLR7-expressing cells are exposed to imiquimod for an extend period of time, such as e.g. 0.5 h - 2h, typically about 1 hour, during the instillation of the inventive pharmaceutical composition, which is sufficient to induce an immunological response exerting the desired therapeutic effect.

Additionally, the pharmaceutical composition of the present invention comprising at least one thermo-sensitive agent, e.g. Poloxamer 407 as defined below, advantageously avoids or at least significantly reduces systemic side effects typically caused by imiquimod. The reduction of systemic side effects is due to the locally restricted administration of imiquimod at the site of affliction, i.e. the bladder, based on increased *in vivo* viscosity of the inventive pharmaceutical composition and the resulting reduced and/or slowed diffusion of the biologically active agent into the surrounding bladder tissue, e.g. the urothelium. In addition, the increased bioadhesive properties of the inventive pharmaceutical composition may contribute to the locally restricted action of imiquimod on the urothelium and thereby on the carcinoma in situ.

Furthermore, the pharmaceutical composition of the present invention comprising at least one thermo-sensitive agent advantageously allow for a so called "extended release" (or sometimes termed "long term release") of imiquimod contained therein. Particularly, the gel formation of the inventive pharmaceutical composition results in a sustained drug release of imiquimod in a zero-order kinetic which enhances the duration of the therapeutic effect. Such prolonged therapeutic effect imiquimod contained in the inventive pharmaceutical composition also avoids repeated administration of the inventive pharmaceutical composition, particularly in short time intervals, which typically cannot be avoided when using pharmaceutical compositions without exhibiting sustained drug release.

Additionally, the LCST of the pharmaceutical composition of the present invention are differently influenced by acetic acid and lactic acid. Both acids generally increase the LCST of the compositions. However, while e.g. imiquimod does not influence at all the gel transition temperature of inventive pharmaceutical compositions comprising only acetic acid, the LCST of compositions comprising lactic acid is enhanced. Thus, the final percentage of thermo-sensitive agents as defined herein may depend on the used acid and whether cyclodextrin is present or not.

The inventive pharmaceutical composition may comprise as the at least one thermo-sensitive agent chitosan or its derivatives, or a poly(ethylene oxide)-poly(propylene oxide)-poly(ethylene oxide) copolymer (also termed PEO-PPO-PEO or poloxamer).

Chitosan in the context of the inventive pharmaceutical composition is typically a linear polysaccharide composed of randomly distributed β-(1-4)-linked D-glucosamine and N-acetyl-D-glucosamine units. Chitosan may be obtained by deacetylation of chitin by treatment with (hot) sodium hydroxide solution. Chitonsan derivatives for use in the inventive pharmaceutical composition may comprise e.g. N-Trimethyl-Chitosan (TMC), Chitosan esters (e.g. glutamate, succinate, phthalate) or chitosan conjugates, such as e.g. chitosan-4- thiobutylamidine.

Poloxamers in the context of the present invention are typically to be understood as a poly(ethylene oxide)-poly(propylene oxide)-poly(ethylene oxide) copolymer, also abbreviated "PEO-PPO-PEO". Such Poloxamers are therefore nonionic triblock copolymers composed of a central hydrophobic chain of polyoxypropylene (poly(propylene oxide)) flanked by two hydrophilic chains of polyoxyethylene (poly(ethylene oxide)). The molecular weight of such Poloxamers is generally not specifically defined and may be varied suitably for each specific purpose. Because the lengths of the polymer blocks can be customized, a multitude of Poloxamers may be provided having slightly different properties. For the generic term "Poloxamer ", these copolymers are commonly named with the letter "P" (for Poloxamer) followed by three digits, the first two digits x 100 give the approximate molecular mass of the polyoxypropylene core, and the last digit x 10 gives the percentage polyoxyethylene content (e.g., P407 = Poloxamer with a polyoxypropylene molecular mass of 4,000 g/mol and a 70% polyoxyethylene content). For the Pluronic/Lutrol tradename, coding of these copolymers starts with a letter to define its physical form at room temperature (L = liquid, P = paste, F = flake (solid)) followed by two or three digits. The first digit (two digits in a three-digit number) in the numerical designation, multiplied by 300, indicates the approximate molecular weight of the hydrophobe; and the last digit x 10 gives the percentage polyoxyethylene content (e.g., L61 = Pluronic with a polyoxypropylene molecular mass of 1,800 g/mol and a 10% polyoxyethylene content). In the example given, Poloxamer 181 (P181) = Pluronic L61.

Poloxamers suitable for the inventive pharmaceutical composition as a thermo-sensitive agent preferably comprise any poly(ethylene oxide)-poly(propylene oxide)-poly(ethylene oxide) copolymer or mixture of such copolymers suitable for the inventive purpose, i.e. any PEO-PPO-PEO polymer or mixture of such copolymers exhibiting thermo-sensitive properties as defined above. Such PEO-PPO-PEO polymers include also commercially available PEO-PPO-PEO polymers and mixtures thereof, e.g. Pluronic F 108 Cast Solid Surfacta; Pluronic F 108 Pastille; Pluronic F 108 Prill; Pluronic F 108NF Prill (Poloxamer 338); Pluronic F 127; Pluronic F 127 Prill; Pluronic F 127 NF; Pluronic F 127 NF 500 BHT Prill; Pluronic F 127 NF Prill (Poloxamer 407); Pluronic F 38; Pluronic F 38 Pastille; Pluronic F 68; Pluronic F 68 Pastille; Pluronic F 68 LF Pastille; Pluronic F 68 NF Prill (Poloxamer 188); Pluronic F 68 Prill; Pluronic F 77; Pluronic F 77 Micropastille; Pluronic F 87; Pluronic F 87 NF Prill (Poloxamer 237); Pluronic F 87 Prill; Pluronic F 88 Pastille; Pluronic F 88 Prill; Pluronic F 98; Pluronic F 98 Prill; Pluronic L 10; Pluronic L 101; Pluronic L 121; Pluronic L 31; Pluronic L 35; Pluronic L 43; Pluronic L 44; Pluronic L 44 NF (Poloxamer 124); Pluronic L 61; Pluronic L 62; Pluronic L 62 LF; Pluronic L 62D; Pluronic L 64; Pluronic L 81; Pluronic L 92; Pluronic L44 NF INH surfactant (Poloxamer 124); Pluronic N 3; Pluronic P 103; Pluronic P 104; Pluronic P 105; Pluronic P 123 Surfactant; Pluronic P 65; Pluronic P 84; Pluronic P 85; and Poloxamer 403. Such PEO-PPO-PEO polymers furthermore include mixtures formed by any two or more (3, 4, 5, 6, etc.) of these PEO-PPO-PEO polymers.

More preferably, Poloxamers suitable for the inventive pharmaceutical composition as a thermo-sensitive agent include Poloxamers or mixtures thereof, the Poloxamers selected from Poloxamer 124, Poloxamer 188, Poloxamer 237, Poloxamer 338, Poloxamer 403, and Poloxamer 407. Thus, using the Poloxamer coding labels of BASF, suitable Poloxamers are selected from Pluronic/Lutrol F 44 (Poloxamer 124), Pluronic/Lutrol F 68 (Poloxamer 188), Pluronic/Lutrol F 87 (Poloxamer 237), Pluronic/Lutrol F 108 (Poloxamer 338), Pluronic/Lutrol F 123 (Poloxamer 403), Pluronic/Lutrol F 127 (Poloxamer 407).

According to an even more preferred embodiment the Poloxamers suitable for the inventive pharmaceutical composition as a thermo-sensitive agent include Poloxamers or mixtures thereof, the Poloxamers selected from Poloxamer 188, Poloxamer 403, and Poloxamer 407.

Even more preferably, such a ratio is selected from a ratio of Poloxamer 407 : Poloxamer 188 of about 1:20, 1:19, 2:18:, 3:17, 4:16, 5:15, 6:14, 7:13, 8:12, 9:11, 10:10 (1:1), 11:9, 12:8, 13:7, 14:6, 15:5, 16:4, 17:3, 18:2, 19:1 or 20:1, or a ratio formed by any of two of the values as defined above, e.g. 1:18, 1:17, 1:16, 1:15, 1:14, 1:13, 1:12, 1:11, 1:10, 1:8, 1:7, 1:6, 1:5, 1:2, or 2:17, 2:15, 2:13, 2:11, 2:9, 2:7, 2:5, 2:3, 2:1, or 3:16, 3:14, 3:13, 3:11, 3:10, 3:8, 3:7, 3:5, 3:4, or 4:15, 4:13, 4:11, 4:9, 4:7, 4:5, or 5:15, 5:13, 5:12, 5:11, 5:9, 5:8, 5:7, 5:6, 5:2, 5:1, or 6:13, 6:11, 6:9, 6:7, 6:5, 6:1, or 7:12, 7:11, 7:10, 7:9, 7:8, 7:6, 7:5, 7:4, 7:3, 7:2, 7:1, or 8:11, 8:9, 8:7, 8:5, 8:3, 8:1, or 9:10, 9:8, 9:7, 9:5, 9:4, 9:2, 9:1, or 10:1, 10:3, 10:7, 10:9, or 11:8, 11:7, 11:6, 11:5, 11:4, 11:3, 11:2, 11:1, or 12:7, 12:5, 12:1, or 13:6, 13:5, 13:4, 13:3, 13:2, 13:1, or 14:5, 14:3, 14:2, 14:1, or 15:4, 15:2, 15:1, or 16:3, 16:1, or 17:2, 17:1, or 18:1, most preferably, such a ratio is selected from a ratio of Poloxamer 407 : Poloxamer 188 of about 7:3, 7.5:2.5, 8:2, 8.5:1.5, 9:1, or 9.5:0.5, or a ratio formed by any of two of these values, e.g. 7:0.5, 7:1, 7:1.5, 7:2, 7:2.5, or 7.5:0.5, 7.5:1, 7.5:1.5, 7.5:2, or 8:2.5, 8:1.5, 8:1. 8:0.5, or 8.5:0.5, 8.5:1.0, or 9.0:0.5. Accordingly, the absolute content of Poloxamer 188 and of Poloxamer 407 in the inventive pharmaceutical composition may be determined on basis of the overall amount and on the specific ratio of both poloxamers in the inventive pharmaceutical composition.

For example, the inventive pharmaceutical composition may comprise as a thermo-sensitive agent a mixture of Poloxamer 407 and Poloxamer 188 in an overall amount of about 15 % (w/v)/(w/w) to about 27.5 % (w/v)/(w/w), more preferably in an overall amount of about 15 % (w/v)/(w/w), of about 20 % (w/v)/(w/w), of about 25 % (w/v)/(w/w) and preferably in a ratio of Poloxamer 407 : Poloxamer 188 of about 15:5, 16:4, 17:3, 18:2, 19:1 or 20:1, or a ratio formed by any of two of these values, more preferably in a ratio of about 9.5:0.5, of about 9:1, of about 8.5:1.5, or of about 8:2, or a ratio formed by any of two of these values. Accordingly, when the inventive pharmaceutical composition comprises a mixture of Poloxamer 407 and Poloxamer 188, Poloxamer 407 may be present in the inventive pharmaceutical composition in an amount of about 15% (w/v)/(w/w) to about 22.5% (w/v)/(w/w), in an amount of about 15.5 % (w/v)/(w/w) to about 26.5 % (w/v)/(w/w), preferably in an amount of about 17.5 % (w/v)/(w/w) to about 22.5 % (w/v)/(w/w), whereas Poloxamer 188 may be present in the pharmaceutical composition in an amount of about 1.0 % (w/v)/(w/w) to about 6.0 % (w/v)/(w/w), preferably in an amount of about 2.5 % (w/v)/(w/w) to about 4.5 % (w/v)/(w/w). The term (w/v)/(w/w) means either (w/v) or (w/w).

Among the different PEO-PPO-PEO polymers suitable for the inventive pharmaceutical composition as a thermo-sensitive agent, Poloxamer 407 is most preferably selected and represents the first choice-polymer for the production of thermo-responsive gels within the context of the present invention. Poloxamer 407 as a thermo-sensitive agent may be used either alone or in admixture with other Poloxamers as described above, preferably with Poloxymer 188, to produce a mixture of thermo-sensitive agents agents that "jellify" at a selected temperature, preferably slightly above room temperature (> 20°C), but below the body temperature (< 37°C).

Pharmaceutical compositions according to the present invention comprising a thermo-sensitive agent are particularly advantageous. For instance, intravesical application of pharmaceutical compositions according to the present invention comprising a thermo-sensitive agent, such as for instance, Poloxamer 407 avoids systemic absorption, and, in addition, provides an increase of the local contact of imiquimod to the urothelium. Thus, addition of a thermo-sensitive agent reduces systemic absorption of imiquimod from bladder urothelium with sustained local infiltration of immune cells.

According to a particularly preferred embodiment, the inventive pharmaceutical composition comprises as a thermo-sensitive agent Poloxamer 407 in an amount as defined above in general for thermo-sensitive agents, more preferably in an amount of about 0.1, 1, 2, 3, 4, 5, 6, 7, 8, 9 or 10 % (w/v) to about 40 % (w/v), or in an amount of of about 2 % (w/v) to about 35 % (w/v), even more preferably in an amount of about 2 % (w/v) to about 30 % (w/v), and most preferably in an amount of about 10 % (w/v) to about 25 % (w/v), e.g. in an amount of about 10.5 % (w/v) to about 25 % (w/v), in an amount of about 11 % (w/v) to about 25 % (w/v), in an amount of about 11.5 % (w/v) to about 25 % (w/v), in an amount of about 12 % (w/v) to about 25 % (w/v), in an amount of about 12.5 % (w/v) to about 25 % (w/v), in an amount of about 13 % (w/v) to about 25 % (w/v), in an amount of about 13.5 % (w/v) to about 25 % (w/v), in an amount of about 14 % (w/v) to about 25 % (w/v), in an amount of about 14.5 % (w/v) to about 25 % (w/v), in an amount of about 15 % (w/v) to about 25 % (w/v), in an amount of about 15.5 % (w/v) to about 25 % (w/v), in an amount of about 16 % (w/v) to about 25 % (w/v), in an amount of about 16.5 % (w/v) to about 25 % (w/v), in an amount of about 17 % (w/v) to about 25 % (w/v), in an amount of about 17.5 % (w/v) to about 25 % (w/v), in an amount of about 18 % (w/v) to about 25 % (w/v), in an amount of about 18.5 % (w/v) to about 25 % (w/v), in an amount of about 19 % (w/v) to about 25 % (w/v), in an amount of about 19.5% (w/v) to about 25 % (w/v), in an amount of about 20 % (w/v) to about 25 % (w/v), in an amount of about 20.5 % (w/v) to about 25 % (w/v), in an amount of about 21 % (w/v) to about 25 % (w/v), in an amount of about 21.5 % (w/v) to about 25 % (w/v), in an amount of about 22 % (w/v) to about 25 % (w/v), in an amount of about 22.5 % (w/v) to about 25 % (w/v), in an amount of about 23 % (w/v) to about 25 % (w/v), in an amount of about 23.5 % (w/v) to about 25 % (w/v), in an amount of about 24 % (w/v) to about 25 % (w/v), or in an amount of about 24.5 % (w/v) to about 25 % (w/v), or more particularly in an amount of about 12 % (w/v) to about 24 % (w/v), in an amount of about 12 % (w/v) to about 23 % (w/v), in an amount of about 12 % (w/v) to about 22 % (w/v), in an amount of about 12 % (w/v) to about 21 % (w/v), in an amount of about 12 % (w/v) to about 20 % (w/v), in an amount of about 12 % (w/v) to about 19 % (w/v), in an amount of about 12 % (w/v) to about 18 % (w/v), in an amount of about 12 % (w/v) to about 17 % (w/v), in an amount of about 12 % (w/v) to about 16 % (w/v), or more particularly in an amount of about 13 % (w/v) to about 24 % (w/v), in an amount of about 13 % (w/v) to about 23 % (w/v), in an amount of about 13 % (w/v) to about 22 % (w/v), in an amount of about 13 % (w/v) to about 21 % (w/v), in an amount of about 13 % (w/v) to about 20 % (w/v), in an amount of about 13 % (w/v) to about 19 % (w/v), in an amount of about 13 % (w/v) to about 18 % (w/v), in an amount of about 13 % (w/v) to about 17 % (w/v), in an amount of about 13 % (w/v) to about 16 % (w/v), or more particularly in an amount of about 14 % (w/v) to about 24 % (w/v), in an amount of about 14 % (w/v) to about 23 % (w/v), in an amount of about 14 % (w/v) to about 22 % (w/v), in an amount of about 14 % (w/v) to about 21 % (w/v), in an amount of about 14 % (w/v) to about 20 % (w/v), in an amount of about 14 % (w/v) to about 19 % (w/v), in an amount of about 14 % (w/v) to about 18 % (w/v), in an amount of about 14 % (w/v) to about 17 % (w/v), in an amount of about 14 % (w/v) to about 16 % (w/v), or more particularly in an amount of about 15 % (w/v) to about 24 % (w/v), in an amount of about 15 % (w/v) to about 23 % (w/v), in an amount of about 15 % (w/v) to about 22 % (w/v), in an amount of about 15 % (w/v) to about 21 % (w/v), in an amount of about 15 % (w/v) to about 20 % (w/v), in an amount of about 15 % (w/v) to about 19 % (w/v), in an amount of about 15 % (w/v) to about 18 % (w/v), in an amount of 16 % (w/v) to about 18% (w/v), in an amount of about 15 % (w/v) to about 17 % (w/v), most preferably in an amount of about 15 % (w/v) to about 16 % (w/v), wherein the amounts as defined in % (w/v) may either be understood to be based on the weight of the thermo-sensitive agent as defined herein with respect to the total volume of the inventive pharmaceutical composition or an intermediate stock solution, when e.g. provided as a liquid or semi-liquid formulation. Alternatively, the above amounts may be defined in % (w/w), wherein the amount as defined in % (w/w) may either be understood to be based on the weight of the thermo-sensitive agent as defined herein with respect to the total weight of the inventive pharmaceutical composition.

Accordingly, the inventive pharmaceutical composition may thus comprise as a thermo-sensitive agent Poloxamer 407 in an (overall) amount of about 10 % (w/v)/(w/w) to about 25.0 % (w/v)/(w/w), more preferably in an overall amount of about 11 % (w/v)/(w/w) to about 22.5 % (w/v)/(w/w), of about 12.0 % (w/v)/(w/w) to about 25.0 % (w/v)/(w/w), of about 12.0 % (w/v)/(w/w) to about 22.5 % (w/v)/(w/w), of about 13 % (w/v)/(w/w) to about 22.5 % (w/v)/(w/w), of about 14.0 % (w/v)/(w/w) to about 22.5 % (w/v)/(w/w), of about 15.0 % (w/v)/(w/w) to about 25.0 % (w/v)/(w/w), of about 15.0 % (w/v)/(w/w) to about 22.5 % (w/v)/(w/w), of about 15.5 % (w/v)/(w/w) to about 22.5 % (w/v)/(w/w), of about 16.0 % (w/v)/(w/w) to about 22.5 % (w/v)/(w/w), or of about 11 % (w/v)/(w/w) to about 20.0 % (w/v)/(w/w), of about 12.0 % (w/v)/(w/w) to about 20.0 % (w/v)/(w/w), of about 13 % (w/v)/(w/w) to about 20.0 % (w/v)/(w/w), of about 14.0 % (w/v)/(w/w) to about 20.0 % (w/v)/(w/w), of about 15.0 % (w/v)/(w/w) to about 20.0 % (w/v)/(w/w), of about 15.5 % (w/v)/(w/w) to about 20.0 % (w/v)/(w/w), of about 16.0 % (w/v)/(w/w) to about 20.0 % (w/v)/(w/w), or of about 11 % (w/v)/(w/w) to about 19.0 % (w/v)/(w/w), of about 12.0 % (w/v)/(w/w) to about 19.0 % (w/v)/(w/w), of about 13 % (w/v)/(w/w) to about 19.0 % (w/v)/(w/w), of about 14.0 % (w/v)/(w/w) to about 19.0 % (w/v)/(w/w), of about 15.0 % (w/v)/(w/w) to about 19.0 % (w/v)/(w/w), of about 15.5 % (w/v)/(w/w) to about 19.0 % (w/v)/(w/w), of about 16.0 % (w/v)/(w/w) to about 19.0 % (w/v)/(w/w), or of about 11 % (w/v)/(w/w) to about 18.0 % (w/v)/(w/w), of about 12.0 % (w/v)/(w/w) to about 18.0 % (w/v)/(w/w), of about 13 % (w/v)/(w/w) to about 18.0 % (w/v)/(w/w), of about 14.0 % (w/v)/(w/w) to about 18.0 % (w/v)/(w/w), of about 15.0 % (w/v)/(w/w) to about 18.0 % (w/v)/(w/w), of about 15.5 % (w/v)/(w/w) to about 18.0 % (w/v)/(w/w), of about 16.0 % (w/v)/(w/w) to about 18.0 % (w/v)/(w/w), or of about 11 % (w/v)/(w/w) to about 17.0 % (w/v)/(w/w), of about 12.0 % (w/v)/(w/w) to about 17.0 % (w/v)/(w/w), of about 13 % (w/v)/(w/w) to about 17.0 % (w/v)/(w/w), of about 14.0 % (w/v)/(w/w) to about 17.0 % (w/v)/(w/w), of about 15.0 % (w/v)/(w/w) to about 17.0 % (w/v)/(w/w), of about 15.5 % (w/v)/(w/w) to about 17.0 % (w/v)/(w/w), of about 16.0 % (w/v)/(w/w) to about 17.0 % (w/v)/(w/w), or of about 11 % (w/v)/(w/w) to about 16.5 % (w/v)/(w/w), of about 12.0 % (w/v)/(w/w) to about 16.5 % (w/v)/(w/w), of about 13 % (w/v)/(w/w) to about 16.5 % (w/v)/(w/w), of about 14.0 % (w/v)/(w/w) to about 16.5 % (w/v)/(w/w), of about 15.0 % (w/v)/(w/w) to about 16.5 % (w/v)/(w/w), of about 15.5 % (w/v)/(w/w) to about 16.5 % (w/v)/(w/w). The term (w/v)/(w/w) means either (w/v) or (w/w)) or any range formed by any of two of these values as defined above.

According to one particularly preferred embodiment the pharmaceutical composition of the present invention as defined above, comprises acetic acid and/or lactic acid in a concentration of about 0.025 M to about 0.2 M, preferably in a concentration of 0.05 M to about 0.150 M, imiquimod in an amount of about 0.1 % (w/v) to about 1 % (w/v), cyclodextrins in an amount of about 2 % (w/v) to about 8 % (w/v) and Poloxamer 407 in an amount of 10 % (w/v) to about 25 % (w/v), preferably in an amount of about 12 % (w/v) to about 25 % (w/v).

According to a further particularly preferred embodiment, the inventive pharmaceutical composition as defined above may comprise lactic acid in a concentration of about 0.025 M (corresponding to 0.225 % (w/v)) to about 0.2 M (corresponding to 1.81 % (w/v)), preferably in a concentration of about 0.03 M (corresponding to 0.27% (w/v)) to about 0.15 M (corresponding to 1.36 % (w/v)), of about 0.035 M (corresponding to 0.315 % (w/v)) to about 0.15 M (corresponding to 1.36 % (w/v)), of about 0.04 M (corresponding to 0.36 % (w/v)) to about 0.125 M (corresponding to 1.125 % (w/v)), of about 0.05 M (corresponding to 0.45 % (w/v)) to about 0.100 M (corresponding to 0.9% (w/v)), imiquimod in a concentration of about 0.1 % (w/v) to about 1 % (w/v), preferably in an amount of about 0.2 % (w/v) to about 1% (w/v), of about 0.3 % (w/v) to about 0.9 %(w/v), of about 0.4% (w/v) to about 0.8 % (w/v), of about 0.5 % (w/v) to about 0.7 % (w/v), Poloxamer 407 in an amount of about 12 % (w/w) to about 18 % (w/w), preferably in an amount of about 13 % (w/w) to about 17 % (w/w), of about 14 % (w/w) to about 16 % (w/w), of about 15 % (w/w) to about 16 % (w/w), of about 16 % (w/w) to about 18 % (w/w), hydroxypropyl-β-cyclodextrin in an amount of about 2 % (w/v) to about 8 % (w/v), preferably in an amount of about 3 % (w/v) to about 8%, of about 3.5 % (w/v) to about 7 % (w/v), of about 4 % (w/v) to about 6 % (w/v), of about 4.5 % (w/v) to about 6 % /w/v), of about 5% (w/v) to about 7 % (w/v), of about 5% /w/v) to about 6% (w/v).

In a most preferred embodiment the inventive pharmaceutical composition as defined above comprises about 0.4% (w/w) imiquimod, Poloxamer 407 in an amount of about 16 % (w/w), hydroxypropyl-β-cyclodextrin in an amount of about 5% (w/w) and lactic acid in an amount of about 0.51% (w/w). The inventive pharmaceutical composition may optionally further comprise a vehicle.

Accordingly, the inventive pharmaceutical composition as defined above is an aqueous solution of a pH value of about 3 to about 8, preferably of about 3 to about 7, more preferably of about 3 to about 6, even more preferably of about 3 to about 5, and most preferably a pH-value of about 3.5 to about 5, including a pH-value in a range of about 3.8 to about 4.9, of about 3.8 to about 4.8, of about 3.9 to about 4.7, of about 3.9 to about 4.6, of about 4.0 to about 4.8, of about 4.0 to about 4.7, of about 4.0 to about 4.6, of about 4.0 to about 4.5, of about 4.0 to about 4.4, or 4.1 to about 5.0, of about 4.1 to about 4.9, of about 4.1 to about 4.8, of about 4.1 to about 4.3, of about 4.1 to about 4.2, or most preferred of about 4.1 to about 4.7. The inventive pharmaceutical composition may be prepared and administered in a pH-value as defined above. If necessary, the pH-value may be further adjusted for the specific treatment and administration requirements, e.g. to a more neutral pH-value of about 5, 6, or 7 (pH 5 to 7) for as long the solubility of imiquimod is not affected, e.g. imiquimod does not precipitate or form any kind of aggregate which would interfere with the desired therapeutic effect of the inventive pharmaceutical composition. The pH of the aqueous inventive pharmaceutical composition may be adjusted by titration with a base, such as e.g. sodium hydroxide (NaOH) or any other suitable OH⁻-donor.

According to one further embodiment, the present invention pertains to the use of the inventive pharmaceutical composition as defined above in the manufacture of a medicament for the treatment of carcinoma in situ bladder cancer and/or for use in aiding in the treatment of carcinoma in situ bladder cancer.

The inventive pharmaceutical composition may be administered at least once a week, e.g. once, twice or three times a week.

More specifically, the inventive pharmaceutical composition may be administered for e.g. at least 3 weeks, for at least 4 weeks, for at least 5 weeks, for at least 6 weeks, for at least 7 weeks, for at least 8 weeks, preferably for about 3-12 weeks, for about 4-12 weeks, for about 4-8 weeks, for about 6-12 weeks, even more preferred for about 6-8 weeks. Accordingly, the inventive pharmaceutical composition may be administered for about e.g. 4 weeks (e.g. once, twice or three times a week), for about 5 weeks (e.g. once, twice or three times a week), for about 6 weeks (e.g. once, twice or three times a week), for about 7 weeks (e.g. once, twice or three times a week), for about 8 weeks (e.g. once, twice or three times a week), for about 9 weeks (e.g. once, twice or three times a week), for about 10 weeks (e.g. once, twice or three times a week), for about 11 weeks (e.g. once, twice or three times a week) or for about 12 weeks (e.g. once, twice or three times a week), or for about e.g. 5-12 weeks, or for about 7-11 weeks, or for about 8-12 weeks, or for about 9-12 weeks, or for about 10-12 weeks, or for about 11-12 weeks or for about 3-10 weeks, or for about 4-9 weeks, or for about 5-8 weeks. The treatment regimen will e.g. depend on the severity of tumour disease, the patient's health status, gender, age, side effects, etc.

The pharmaceutical composition according to the invention may preferably be administered at time intervals of about 2-7 days, e.g. of 2, 3, 4, 5, 6 or 7 days, preferably 7 days. Accordingly, the inventive pharmaceutical composition as defined above may be administered at e.g. days 0, 2, 4, 6, 8 and so on, or at days 0, 3, 6, 9, 12, 15 and so on, or at days, 4, 8, 12, 16, 20 and so on, or at days 0, 5, 10, 15 and so on, or days 0, 6, 12, 18, 24 and so on, or days 0, 7, 14, 21, 28 and so on, until the end of the time interval for administration as defined above. Herein, "day 0" denotes the day of the first treatment, e.g. the day on which the first administration, e.g. the first intravesical instillation, of the inventive pharmaceutical composition is carried out. The intravesical instillations of the inventive pharmaceutical composition as defined above may be carried out by any means known to the skilled person suitable for the particular purpose, e.g. by means of a transurethral catheter.

Accordingly, the inventive pharmaceutical composition may be administered for a total of e.g. at least 3 doses, of at least 4 doses, preferably of at least 5 doses, of at least 6 doses, of at least 7 doses or of at least 8 doses, or e.g. of 3-12 doses, of 4-11 doses, of 5-10 doses, of 6-9 doses, of 7-8 doses, or of e.g. 10 doses, 12 doses, 14 doses, of 16 doses, of 18 doses, of 20 doses, of 22 doses, of 24 doses, of 26 doses, of 28 doses, of 30 doses, of 32 doses, of 34 doses, of 36 doses, or e.g. of about 3-36 doses, of about 4-32 doses, of about 6-30 doses, of about 8-28 doses, of about 10-26 doses, of about 12-24 doses, of about 14-20 doses, of about 16-22 doses, of about 18-20 doses, or of e.g. 4 doses, of 5 doses, of 6 doses, of 7 doses, of 8 doses, of 9 doses, of 10 doses, of 11 doses, of 12 doses, of 13 doses, of 14 doses, of 15 doses, of 16 doses, of 17 doses, of 18 doses, of 19 doses, of 20 doses, of 21 doses, of 22 doses, of 23 doses, of 24 doses, of 25 doses, of 26 doses, of 27 doses, of 28 doses, of 29 doses, of 30 doses, of 31 doses, of 32 doses, of 33 doses, of 34 doses, of 35 doses, of 36 doses.

According to a more specific embodiment, the inventive pharmaceutical composition (depending e.g. on the chosen imiquimod concentration, or e.g. bladder volume of the patient) may be administered in a volume of e.g. about 10-100ml, preferably in a volume of e.g. about 20ml, of about 30ml, of about 40ml, of about 50ml, of about 60ml, of about 70ml of about 80ml, of about 90, or in a volume of about 25ml-100ml, of about 30ml-100ml, of about 35ml-100ml, of about 40ml-100ml, of about 45ml-100ml, of about 50ml-100ml, of about 55ml-100ml, of about 60ml-100ml, of about 65ml-100ml, of about 70ml-100ml, of about 75ml-100ml, of about 80ml-100ml, of about 85ml-100ml, of about 90ml-100ml, of about 95ml-100ml, or in a volume of e.g. about 25-50ml, of about 30-55ml, of about 35-60ml, of about 40ml-65ml, of about 45ml-70ml, of about 50ml-75ml, of about 55ml-80ml, of about 60ml-85ml, of about 65ml-90ml, of about 70ml-95ml, more preferably in a volume of about 40ml-70ml, or in a volume of e.g. about 50ml.

The pharmaceutical composition according to the invention is formulated such that the intravesical retention time of the inventive pharmaceutical composition as defined above may be e.g. at least about 0.5h, at least about 1h, at least about 1.5h, at least about 2h, at least about 2.5h, at least about 3h, at least about 3.5h, or of about 4h, preferably between e.g. 0.5 h to about 2h, between about 0.75h to about 2h, between about 1h to about 1.5h, between about 1.5h to about 2h, between about 2h to about 4h, between about 2.5 to about 3h, between about 2.5h to about 3.5h, or e.g. for about 0.6h, for about 0.7h, for about 0.8h, for about 0.9h, for about 1h, for about 1.1h, for about 1.2h, for about 1.3h, for about 1.4h, for about 1.5h, for about 1.6h, for about 1.7h, for about 1.8h, for about 1.9h, for about 2h. Retention time as used in the context of the inventive pharmaceutical composition as defined above shall refer to the intravesical retention time of the inventive pharmaceutical composition, or e.g. may also refer to the time in which e.g. at least 50% (e.g. 60%, 70%) of the imiquimod of the pharmaceutical composition are absorbed by the urothelium.

Without being bound thereto, the inventive pharmaceutical composition will contain or release sufficient active imiquimod to provide a dose of about 10, 20, 50, or 100 nanograms per kilogram (ng/kg) to about 50 milligrams per kilogram (mg/kg), preferably about 10 micrograms per kilogram (µg/kg) to about 5 mg/kg, of imiquimod or a salt thereof to the subject. Accordingly, the inventive pharmaceutical composition will contain or release sufficient imiquimod to provide a dose of, for example, from about 0.0001, 0.001, 0.01 or 0.01 mg/m² to about 5.0 mg/m², computed according to the Dubois method, in which the body surface area of a subject (m²) is computed using the subject's body weight: m² = (wt kg^{0.425} x height cm^{0.725}) x 0.007184, although in some embodiments the methods may be performed by administering a compound or salt or composition in a dose outside this range. In some of these embodiments, the method includes administering sufficient imiquimod to provide a dose of from about 0.0001, 0.001, 0.01, or 0.1 mg/m² to about 2.0 mg/ m² to the subject, for example, a dose of from about 0.004, 0.04, or 0.4 mg/m² to about 1.2 mg/m².

For some embodiments of the present invention one or more of the following provisions apply:
Topical use of the pharmaceutical composition of the present invention is preferably excluded from the scope of the present invention. Furthermore compositions comprising oil, e.g. emulsions are preferably excluded from the present invention, pharmaceutical compositions formulated as a w/o (water-in-oil) or o/w (oil-in-water) formulation are also preferably excluded. Also pharmaceutical compositions formulated as a cream comprising 4 % (w/w) imiquimod in the oil phase and 1 % (w/w) by weight lactic acid (85 %) in the aqueous phase are preferably excluded. Pharmaceutical compositions for parenteral administration are preferably excluded. Pharmaceutical compositions for parenteral administration comprising 1% (w/w) imiquimod and/or 1% or 2 % (w/w) lactic acid (85 %) or 0.6 % (w/w) acetic acid are preferably excluded. Furthermore, pharmaceutical compositions comprising acetic acid and sorbitan monooleate 20 myristate or isopropyl myristate are excluded, and/or pharmaceutical compositions comprising imiquimod chitosan nanoparticels obtained by mixing chitosan acetic acid solution with imiquimod are excluded. Depot formulation as disclosed in US 2004/0265351 A1 are also preferably excluded from the scope of the present invention, e.g. depot formulations, which are retained in or on the human body for longer than 4 hours.

According to one embodiment, the present invention pertains to methods of treatment of carcinoma in situ bladder cancer, wherein the method comprises the steps of administering to a person in need thereof a therapeutically effective amount of the inventive pharmaceutical composition as defined above.

Accordingly, the method comprises the intravesical administration of the inventive pharmaceutical composition as defined above, wherein the inventive pharmaceutical composition may be administered e.g. over 1min - 60min, over 2min-50min, over 3min-45min, over 4min-40min, over 5min-35min, over 10min-30min, over 15min-20min, preferably e.g. over 2min - 15 min, or over 3min - 15 min, or over 4min - 12min, or over 5min - 10min, more preferably over 3 min - 9min, or over 3min - 8min, or 4min - 8 min, most preferably over 3min - 5min. The method further comprises administering the inventive pharmaceutical composition over the course of e.g. at least 4 weeks, of at least 5 weeks, of at least 6 weeks, of at least 7 weeks, of at least 8 weeks, preferably of about 3-12 weeks, of about 4-12 weeks, of about 4-8 weeks, of about 6-12 weeks, even more preferred of e.g. about 6-8 weeks. Accordingly, the method of treatment of the invention may comprise administering the inventive pharmaceutical composition for e.g. about 4 weeks, for about 5 weeks, for about 6 weeks, for about 7 weeks, for about 8 weeks, for about 9 weeks, for about 10 weeks, for about 11 weeks, for about 12 weeks, or for about e.g. 5-12 weeks, or for about 7-11 weeks, or for about 8-12 weeks, or for about 9-12 weeks, or for about 10-12 weeks, or for about 11-12 weeks or for about 3-10 weeks, or for about 4-9 weeks, or for about 5-8 weeks.

Accordingly, the method of treatment of CIS bladder cancer according to the invention may comprise intravesical instillations of the inventive pharmaceutical composition as defined above beginning at day 0 in any of the time intervals as defined above, such as e.g. day 0, 7, 14, 21, 28 and so on as defined above, for e.g. at least 4 weeks, or at least 5 weeks, or at least 6 weeks, or at least 7 weeks, or at least 8 weeks, or for any period of time as defined above.

According to a more specific embodiment, the method of treating CIS bladder cancer of the invention may comprise an intravesical retention time of the inventive pharmaceutical composition of e.g. at least about 0.5h, of at least about 1 h, of at least about 1.5h, of at least about 2h, of at least about 2.5h, of at least about 3h, of at least about 3.5h, at least about 4h, preferably of e.g. between 0.5 h to about 2h, between about 0.75h to about 2h, between about 1h to about 1.5h, between about 1.5h to about 2h, between about 2h to about 4h, between about 2.5 to about 3h, between about 2.5h to about 3.5h, or e.g. of about 0.6h, of about 0.7h, of about 0.8h, of about 0.9h, of about 1 h, of about 1.1h, of about 1.2h, of about 1.3h, of about 1.4h, of about 1.5h, of about 1.6h, of about 1.7h, of about 1.8h, of about 1.9h, of about 2h.

Depending on the severity of the CIS bladder cancer, the treatment regimen may be adapted accordingly, e.g. factors that may influence treatment options may be the age of the patient, severity (stage) of the CIS bladder cancer and the adverse effects experienced during the course of the cancer treatment. For example, the duration of the cancer treatment may be reduced or extended, while increasing the time intervals between individual instillations of the inventive pharmaceutical composition, e.g. from administration intervals of day 0, 3, 6, 9, and so on to e.g. day 0, 5, 10, 15, 20 and so on, or to e.g. day 0, 7, 14, 21, 28 and so on. Likewise, the intravesical retention time as defined above may be reduced or increased depending on CIS bladder cancer severity. Accordingly, the concentration of imiquimod in the inventive pharmaceutical composition may be varied depending on disease severity, e.g. the inventive pharmaceutical composition may comprise any of the amounts of imiquimod as defined above, or as exemplified in the appended examples.

Depending on the severity of the CIS bladder cancer, the patient in need of CIS bladder cancer treatment according to the invention may require more than one treatment cycle, e.g. the patient may require repeating the inventive CIS bladder cancer treatment method as defined above, e.g. at least 2, 3, 4, 5 or 6 times.

Preferably, the pharmaceutical composition according to the present invention is administered in a combination therapy, wherein (i) the pharmaceutical composition according to the present invention is administered to the patient to treat the CIS bladder cancer and (ii) a concurrent exophytic tumour is treated concomitantly. Such a combination therapy is thus particularly useful if the CIS bladder cancer is concurrent CIS bladder cancer. In such a combination therapy, the pharmaceutical composition according to the present invention may be administered as described above and the concurrent exophytic tumour, in particular the papillary urothelial tumor, may be treated concomitantly in particular by surgery and/or pharmaceutically, e.g. by chemotherapy. Preferably, the concurrent exophytic tumour is removed surgically.

In the combination therapy according to the present invention, "concomitant treatment" means that the pharmaceutical composition according to the present invention may be administered before, during or after treatment of the concurrent exophytic tumour. For example, pharmaceutical therapy, e.g. chemotherapy, and/or surgery of the concurrent exophytic tumour may be carried out before, during or after a treatment cycle with the pharmaceutical composition according to the present invention as described above.

Thereby, administration before the treatment of the concurrent exophytic tumour refers to any treatment cycle wherein the last treatment with the pharmaceutical composition according to the present invention is finished before the treatment of the concurrent exophytic tumour begins. Preferably, the last treatment with the pharmaceutical composition according to the present invention is finished no later than 7, 6, 5, 4, 3, 2, or 1 days before the treatment of the concurrent exophytic tumour begins, more preferably, the last treatment with the pharmaceutical composition according to the present invention is finished no later than 48h, 36h, 24h, 20h, 16h, 12h, 8h, or 4h before the treatment of the concurrent exophytic tumour begins, and even more preferably, the last treatment with the pharmaceutical composition according to the present invention is finished no later than 180 min, 120 min, 90 min, 60 min, 45 min, 30 min, 20 min, 10 min, 9 min, 8 min, 7 min, 6 min, 5 min, 4 min, 3 min, 2 min, or 1 min before the treatment of the concurrent exophytic tumour begins.

Thereby, administration after the treatment of the concurrent exophytic tumour refers to any treatment cycle wherein the first treatment with the pharmaceutical composition according to the present invention begins after the treatment of the concurrent exophytic tumour is finished. Preferably, the first treatment with the pharmaceutical composition according to the present invention begins no later than 7, 6, 5, 4, 3, 2, or 1 days after the treatment of the concurrent exophytic tumour is finished, more preferably, the first treatment with the pharmaceutical composition according to the present invention begins no later than 48h, 36h, 24h, 20h, 16h, 12h, 8h, or 4h after the treatment of the concurrent exophytic tumour is finished, and even more preferably, the first treatment with the pharmaceutical composition according to the present invention begins no later than 180 min, 120 min, 90 min, 60 min, 45 min, 30 min, 20 min, 10 min 9 min, 8 min, 7 min, 6 min, 5 min, 4 min, 3 min, 2 min, or 1 min after the treatment of the concurrent exophytic tumour is finished.

Thereby, administration during the treatment of the concurrent exophytic tumour refers to any treatment cycle with the pharmaceutical composition according to the present invention which overlaps with the treatment of the concurrent exophytic tumour. Accordingly, administration (of the pharmaceutical composition according to the present invention) during the treatment of the concurrent exophytic tumour is particularly preferred in the combination therapy according to the present invention.

It is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### EXAMPLES

### Example 1

Examples for liquid formulations of pharmaceutical compositions according to the present invention, which comprise imiquimod for use in the treatment of carcinoma in situ of the bladder:

| **Amount imiquimod (w/w)** | | **0.1%** | **0.2%** | **0.4%** |
|---|---|---|---|---|
| **Ingedient** | **Function** | **Quantity (g/l)** | **Quantity (g/l)** | **Quantity (g/l)** |
| Imiquimod | Active ingredient | 1.00 | 2.00 | 4.00 |
| Poloxamer 407 | Emulsifying agent | 160.00 | 160.00 | 160.00 |
| Hydroxypropylbetadex | Stabilizing agent | 50.00 | 50.00 | 50.00 |
| Lactic acid (50% (w/w)) | Solvent | 10.2 | 10.2 | 10.2 |
| NaOH solution | pH adjusting agent | q.s. to pH 4.1 - 4.7 | q.s. to pH 4.1 - 4.7 | q.s. to pH 4.1 - 4.7 |
| Water for injection | Solvent (vehicle) | q.s. to 1l | q.s. to 1l | q.s. to 1l |

The above liquid formulations are for intravesical administration, wherein individual doses typically have a volume of about e.g. 50ml. The liquid formulations are administered via a catheter into the bladder of a patient over a period of time of typically about 3-5 minutes. The retention time of the (semi)liquid formulation in the bladder of a patient is typically about 1 hour.

The above pharmaceutical compositions are given for a total of 6 weeks, wherein the treatment regimen comprises administration of the pharmaceutical composition at day 0, 7, 14, 21, 28 and 35 during the 6 week interval. "Day 0" denotes the day of the first intravesical instillation of the inventive pharmaceutical composition.

### Example 2

Phase II pilot study with imiquimod in patients with carcinoma in situ (CIS) bladder cancer

Objectives of the study are the assessment of imiquimod activity in the treatment of CIS bladder cancer by the number of patients who experience complete response (CR). Herein, CR is defined as no evidence of disease (negative histology, negative cytology) 5 to 7 weeks after the last imiquimod instillation. Furthermore, the safety and the tolerability of imiquimod administered once weekly for 6 weeks was assessed, as measured by pharmacodynamic markers in urine and in pre- and post-dosing tumour biopsies.

### Study design:

The study is an open-label, pilot phase II study investigating the response to intravesical imiquimod in patients with CIS bladder cancer. 12 patients are to be enrolled at multiple (4) study centers in the United States in order to obtain 6 evaluable patients. Enrollment will be ended once the sixth patient becomes evaluable, or when a total of 12 patients has been admitted into the study, whichever occurs first. All patients enrolled at this time will be allowed to complete the study.

Patients who have been diagnosed with CIS bladder cancer are potential candidates for the study. The CIS lesion(s) may be primary, secondary, or recurrent, and may be concomitant to a Ta or T1 lesion, provided the Ta or T1 lesion has been fully resected.

After provision of written informed consent, patients are to be evaluated for study eligibility during the screening period within 28 days (4 weeks) before Day 0 (i.e., the first day of study drug administration). Screening evaluations to be performed include medical history, physical examination, vital signs, Eastern Cooperative Oncology Group (ECOG) performance status, hematology and clinical chemistry, and urinalysis with culture. Screening evaluations also include a cystoscopic examination of the bladder with mapping and biopsy/ies of all suspicious areas between Days -28 and -14, followed by a bladder washing for cytology. Patients who have undergone such examinations as part of the diagnostic process outside of the study are not required to undergo the procedures again, provided the examinations occurred within the specified timeframe and positive slides are available for a central pathology and cytology review.

Patients for whom CIS bladder cancer is confirmed by histology who fulfill all other eligibility criteria will be enrolled in the study on Day 0, the first day of study drug administration. All patients will receive imiquimod 0.4% (w/w) in a volume of 50 mL (200 mg in 50 mL) once weekly for 6 weeks (on Days 0, 7, 14, 21, 28, and 35), for a total of 6 doses. During treatment, patients will receive imiquimod at the study center and will have study evaluations performed on Days 7, 14, 21, 28, and 35.

Patients are to attend a follow-up study center visit 5 to 7 weeks after the last imiquimod dose for cytology to be performed and biopsies to be collected. In cases with no visible lesions, at least one biopsy will be taken at the prior positive site and at least 1 additional random biopsy will be taken. Response to treatment will be determined based on urine cytology and tissue sample histology findings.

### Number of Patients

12 patients will be enrolled in order to obtain 6 evaluable patients (i.e., patients who had positive histology for CIS of the bladder during Screening, received all 6 imiquimod doses, and had a cystoscopic examination of the bladder with bladder washing 5 to 7 weeks after the last imiquimod dose, with readable histology and cytology samples obtained).

### Diagnosis and main criteria for inclusion

Patients meeting all of the following criteria were considered eligible for study entry:
1. Males or female patient is aged ≥18 years.
2. Patient has pathologically-proven, recurrent, primary, secondary, or concomitant carcinoma in situ disease, defined by having either CIS alone or Ta or T1 with CIS, provided the Ta or T1 lesion(s) has/have been fully resected. In cases with T1 tumor lesions, muscularis propria tissue should be in the resected specimen to confirm that it is tumor-free.
3. Patient has undergone mapping of the bladder between Days -28 and -14, with at least one biopsy providing pathological confirmation of CIS of the bladder.
4. Patient has undergone bladder washing for cytology between Days -28 and -1. In patients in whom Ta or T1 lesion(s) were resected, the bladder washing must have occurred after the resection.
5. Patient has an ECOG performance status 0-2.
6. Patient has adequate bone marrow, hepatic, and renal function within 4 weeks before Day 0.

Patients showing e.g. evidence of muscle-invasive disease (i.e., T2 grade bladder cancer or higher) or were reported not to be able to hold instillation for at least 1 hour, or patients of being suspected of being hypersensitive to imidazoquinoline compounds, Poloxamer 407, hydroxy propyl betacyclodextrin, or lactic acid were not included in the study.

### Test products, dosing and mode of administration

Imiquimod was supplied in plastic bags containing 50 mL of a sterile colorless liquid solution stable at room temperature and ready to be instilled in the bladder. Plastic bags were provided with a urologic connector and were wrapped with an aluminum quadruplex foil. All patients received the same volume of 50 mL and the same dose of 0.4% (w/v) imiquimod (200 mg imiquimod in 50 mL). Bags containing imiquimod 0.2% were also supplied in the event dose reductions were needed.

Imiquimod was administered intravesically by gravity over 3 to 5 minutes through a standard catheter. If gravity was not sufficient for proper instillation, the Investigator was allowed to squeeze the bag gently to instill imiquimod over 3 to 5 minutes. The retention time was supported to be 1 hour. Patients received intravesical imiquimod 0.4% once weekly for 6 weeks.

### Safety

Safety was determined by documentation of adverse events (AEs), clinical laboratory tests (hematology, clinical chemistry, urinalysis, and urine culture), physical examinations, and vital sign measurements.

### Efficacy

Efficacy was evaluated by determination of clinical benefit to patients, defined as a complete response (CR) to treatment 5 to 7 weeks after the imiquimod dose. Determination of CR was made based on urine cytology and tissue sample histology findings.

### Results

12 patients were enrolled in the study, representing a heterogenous patient population with CIS with both treatment-naïve and heavily pre-treated patients. Out of the 12 patients enrolled, 8 completed the treatment, while for 4 patients the treatment was ongoing. 5 patients were evaluated with a further of 7 patients who have not been subjected to follow-up evaluation, the results of which are as follows:

### Patient 1

Patient 1, who exhibited with CIS / high grade papillary bladder cancer in November 2012 and CIS in March of 2013, received BCG treatment from December 2012 to January 2013. Prior to the treatment with imiquimod, the patient presented histologically, with local and central CIS. Following the imiquimod treatment regimen according to the study design, no tumor was detecable by histology. Preliminiary results indicate that no tumour was detectable in that patient in follow-up evaluation.

### Patient 2

Patient 2 who presented with low grade papillary bladder cancer as well as high grade stage cancer since 2008 and with carcinoma in situ in 2011. The patient received chemotherapy, BCG and 9x TURBT. Histologically, local CIS was detected. Following imiquimod treatment according to the study design, there was still CIS detected, however the ratio of malignant to non-malignant cells seems to be improved. This patient might be considered as a partial responder. Further evaluation is still ongoing.

### Patient 3

Patient 3 was diagnosed with bladder cancer in February of 2013 and had not received any previous cancer treatment, prior to study enrollment. Histologically, patient 3 exhibited TIS, papillary high and low grade cancer as well as CIS and papillary low and high grade cancer. Following the treatment with imiquimod according to the study design, no tumor was detected any more either by cytology or histology.

### Patient 5

Patient 5 presented with CIS in 2011 and CIS in 2013. Previous anti-cancer treatment comprised BCG and mitomycin treatment. Histologically, the patient exhibited CIS bladder cancer. Following the treatment with imiquimod according to the study design, no tumor was detectable by histology.

In summary the results show that the predominantly male CIS bladder cancer patients of the above study, when treated with the inventive pharmaceutical composition according to Example 1 (see above), exhibit a significant health improvement, i.e. tumor regression: Of the 5 patients analyzed, 4 unsuccessfully underwent prior cancer treatment (see table 2, patients 1, 2, 4, 5). Four patients from this group did exhibit complete remission of the CIS bladder cancer as assessed by histology (see: Table 2, patients 1, 3, 4, 5). Table 2 summarizes the results of the imiquimod study.

**Table 1: Anti-cancer pre-treatment of the patients enrolled in the sudy**

| **Patient number** | **Site** | **Prior Chemotherapy** | **Age/Gender** | **Prior Immunotherapy** | **Prior TURB** | **CIS first diagnosis** | **CIS WHO grade at study entry 2013** |
|---|---|---|---|---|---|---|---|
| 01 | 1 | 0 | 61/M | BCG | 3 | 07. Nov 2012 | 4° |
| 02 | 2 | Mitomycine C Gemcitabine, Docetaxel, Doxirubicin | 76/M | BCG | 9 | Dec 2008 | 4° |
| 03 | 3 | 0 | 54/F | 0 | 0 | 15. Feb 2013 | 3° |
| 04 | 1 | 0 | 66/M | BCG | 1 | 03. Apr 2013 (1989 "bladder tumor") | 4° |
| 05 | 2 | Mitomycine C | 64/M | BCG | 0 | Mar 2011 | 4° |
| 06 | 4 | 0 | 62/M | 0 | 0 | Jul 2013 (May-2011 Papillary TU) | 3° |
| 07 | 3 | Mitomycin | 73/M | 0 | 2 | May 2011 | 3° |
| 08 | 3 | Intravesical valstarX2; valstar+BCG treatmentX2 | 80/M | BCG | 8 (since 1996) | 1986: Bladder cancer unkown type May-2013 (Recurrent transitional cell cancer of the bladder) | 4° (2013) |
| 09 | 1 | 0 | | 0 | 3 | 2013 | |
| 10 | 1 | 0 | | 0 | 0 | Aug 2013 | |
| 11 | 4 | 0 | | BCG (6 x) | 3 | 10. Sep 2004 | na (entry 10/2013) |
| 12 | 1 | NA | | ? | | 2012 | |

| | | | | | | | |
|---|---|---|---|---|---|---|---|
| No data available from patients 09, 10, 12 at the time of filing. "NA" denotes insecure data or data not available. Gender "M": Male, "F" female | | | | | | | |

**Table 2: Summary of the study results of the imiquimod study.**

| **Pat.** | **History** | **Prior treatment** | **Histology prior to treatment** | **Histology post treatment** | **Summary^{#}** |
|---|---|---|---|---|---|
| 1 | Diagnosed 2012 | BCG | CIS | No tumor | BCG refractory Responder |
| 2 | Since 2008 recurent low, high grade, 2011 CIS | Chemo, BCG | CIS | CIS | partial responder |
| 3 | Feb. 2013 | 0 | CIS + papillary high and low grade cancer | No tumor | Responder |
| 4 | Bladder tumor in 1989(?) and in 2013 | TURB | Low grade | No tumor | Excluded^{§} |
| 5 | CIS in 2011 and 2013 | BCG/Mitomycin | CIS | No tumor | Responder |

| | | | | | |
|---|---|---|---|---|---|
| "^{?}" denotes insecure data or data not available ^{#}"responder" denotes patients that have responded to the inventive CIS bladder cancer treatment, "non-responder" refers to patients in which the inventive cancer treatment did not result in remission of CIS bladder cancer. ^{§} Histological results indicated that patient 4 had no CIS bladder cancer and was thus to be excluded from the study. | | | | | |

### ITEMS

1. Pharmaceutical composition comprising imiquimod for use in the treatment of carcinoma in situ (CIS) of the bladder.
2. Pharmaceutical composition for use according to item 1, wherein the carcinoma in situ (CIS) of the bladder is selected from the group consisting of primary carcinoma in situ (CIS) of the bladder, secondary carcinoma in situ (CIS) of the bladder and concurrent carcinoma in situ (CIS) of the bladder.
3. Pharmaceutical composition for use according to item 2, wherein the carcinoma in situ (CIS) of the bladder is concurrent carcinoma in situ (CIS) of the bladder.
4. Pharmaceutical composition for use according any one of items 1 - 3, further comprising at least one pharmaceutically acceptable excipient and optionally a vehicle.
5. Pharmaceutical composition for use according any one of items 1 - 4 comprising imiquimod in an amount of about 0.005 % (w/v) to about 2 % (w/v), including an amount of about 0.01 % (w/v) to about 2 % (w/v), an amount of about 0.1 % (w/v) to about 1.5 % (w/v), an amount of about 0.1 % (w/v) to about 1 % (w/v), an amount of about 0.2 % (w/v) to about 0.9 % (w/v), an amount of about 0.3 % (w/v) to about 0.9 % (w/v), an amount of about 0.4 % (w/v) to about 0.8 % (w/v), an amount of about 0.5 % (w/v) to about 1 % (w/v) or an amount of about 0.2 % (w/v) to about 1 % (w/v), preferably in an amount of about 0.1 % (w/v) to about 0.5 (w/v).
6. Pharmaceutical composition for use according to any one of items 1-5 for use in the treatment of a CIS bladder cancer patient in the age of 40 years or older, preferably in the age of 60 years and older.
7. Pharmaceutical composition for use according to any one of items 1-6, wherein the CIS bladder cancer patient is male.
8. Pharmaceutical composition for use according to any one of items 1-7, wherein the CIS bladder cancer patients exhibits at least 2 CIS bladder tumour sites in the bladder mucosa.
9. Pharmaceutical composition for use according to any of items 1-8, further comprising at least one organic acid.
10. Pharmaceutical composition for use according to any of items 1-9, comprising acetic acid and/or lactic acid in a concentration of about 0.025 M to about 0.200 M, preferably in a concentration of about 0.025 M to about 0.100 M, or in a concentration of about 0.035 M to about 0.1 M, or in a concentration of about 0.045 M to about 0.1 M, or in a concentration of about 0.055 M to about 0.1 M, or in a concentration of about 0.065 M to about 0.1 M, or in a concentration of about 0.075 M to about 0.1 M, or in a concentration of about 0.085 M to about 0.1 M, or in a concentration of about 0.100 M to about 0.200 M, or in a concentration of about 0.100 M to 0.150 M, or in a concentration of about 0.075 to about 0.200 M
11. Pharmaceutical composition for use according to any of items 1-10 further comprising at least one thermo-sensitive agent.
12. Pharmaceutical composition for use according to any of items 1-11, wherein the at least one thermo-sensitive agent exhibits a specific "lower critical solution temperature" (LCST) in a range of about 15°C to about 35°C, more preferably in a range of about 15°C to about 30°C, even more preferably in a range of about 15 or 20°C or 25°C to about 30°C, most preferably in a range of about 15 or 20°C to about 25°C.
13. Pharmaceutical composition for use according to any of items 1-12, comprising the least one thermo-sensitive agent in an amount of about 0.1 % (w/v) to about 40 % (w/v), typically in an amount of about 2 % (w/v) to about 30 % (w/v), preferably in an amount of about 5 % (w/v) to about 30 % (w/v), more preferably in an amount of about 10 % (w/v) to about 30 % (w/v), and most preferably in an amount of about 10 % (w/v) to about 25 % (w/v) or in an amount of about 10 % (w/v) to about 20 % (w/v).
14. Pharmaceutical composition for use according to any of items 1-13, wherein the at least one thermo-sensitive agent is selected from a poly(ethylene oxide)-poly(propylene oxide)-poly(ethylene oxide) copolymer (also termed PEO-PPO-PEO or poloxamer) or from chitosan or its derivatives.
15. Pharmaceutical composition for use according to any of items 1-14, wherein the at least one thermo-sensitive agent is selected from a poly(ethylene oxide)-poly(propylene oxide)-poly(ethylene oxide) copolymer (also termed PEO-PPO-PEO or poloxamer).
16. Pharmaceutical composition for use according to any one of items 1-15, wherein the at least one thermo-sensitive agent is selected from a poly(ethylene oxide)-poly(propylene oxide)-poly(ethylene oxide) copolymer (also termed PEO-PPO-PEO or poloxamer) including Pluronic F 108 Cast Solid Surfacta; Pluronic F 108 Pastille; Pluronic F 108 Prill; Pluronic F 108NF Prill (Poloxamer 338); Pluronic F 127; Pluronic F 127 Prill; Pluronic F 127 NF; Pluronic F 127 NF 500 BHT Prill; Pluronic F 127 NF Prill (Poloxamer 407); Pluronic F 38; Pluronic F 38 Pastille; Pluronic F 68; Pluronic F 68 Pastille; Pluronic F 68 LF Pastille; Pluronic F 68 NF Prill (Poloxamer 188); Pluronic F 68 Prill; Pluronic F 77; Pluronic F 77 Micropastille; Pluronic F 87; Pluronic F 87 NF Prill (Poloxamer 237); Pluronic F 87 Prill; Pluronic F 88 Pastille; Pluronic F 88 Prill; Pluronic F 98; Pluronic F 98 Prill; Pluronic L 10; Pluronic L 101; Pluronic L 121; Pluronic L 31; Pluronic L 35; Pluronic L 43; Pluronic L 44; Pluronic L 44 NF (Poloxamer 124); Pluronic L 61; Pluronic L 62; Pluronic L 62 LF; Pluronic L 62D; Pluronic L 64; Pluronic L 81; Pluronic L 92; Pluronic L44 NF INH surfactant (Poloxamer 124); Pluronic N 3; Pluronic P 103; Pluronic P 104; Pluronic P 105; Pluronic P 123 Surfactant; Pluronic P 65; Pluronic P 84; Pluronic P 85; and Poloxamer 403, or is selected from a mixture formed by any two or more of the afore defined thermo-sensitive agents.
17. Pharmaceutical composition for use according to any of items 1-16, wherein the at least one thermo-sensitive agent is Poloxamer 407 and/or Poloxamer 188 or a mixture of Poloxamer 407 and Poloxamer 188.
18. Pharmaceutical composition for use according to any one of items 1-17, wherein the at least one thermo-sensitive agent is a mixture of Poloxamer 407 and Poloxamer 188 in an (overall) amount of about 22.5 % (w/v)/(w/w) to about 27.5 % (w/v)/(w/w), more preferably in an overall amount of about 25 % (w/v)/(w/w), and in a ratio of Poloxamer 407 : Poloxamer 188 of about 15:5, 16:4, 17:3, 18:2, 19:1 or 20:1, or a range formed by any of two of these ratio values, more preferably in a ratio of about 9.5:0.5, of about 9:1, of about 8.5:1.5, or of about 8:2, or a range formed by any of two of these ratio values.
19. Pharmaceutical composition for use according to any one of items 1-18, wherein the at least one thermo-sensitive agent is Poloxamer 407 in an amount of about 10 % (w/v) to about 25 % (w/v) or in an amount of about 12 % (w/v) to about 25 % (w/v), or in an amount of 14 % (w/v) to about 25 % (w/v), or in an amount of 15 % (w/v) to about 20% (w/v).
20. Pharmaceutical composition for use according to any one of item 1-19, wherein the at least one thermo-sensitive agent is Poloxamer 407 in an (overall) amount of about 15.0 % (w/v)/(w/w) to about 22.5 % (w/v)/(w/w), preferably in an (overall) overall amount of about 15.5 % (w/v)/(w/w) to about 20 % (w/v)/(w/w), more preferably in an amount of about 16 % (w/v)/(w/w) to about 22.5 % (w/v)/(w/w), of about 16.5 % (w/v)/(w/w), of about 17.0 % (w/v)/(w/w), of about 17.5 % (w/v)/(w/w), of about 18.0 % (w/v)/(w/w), of about 18.5 % (w/v)/(w/w), of about 19.0 % (w/v)/(w/w), of about 19.5 % (w/v)/(w/w), of about 20.0 % (w/v)/(w/w), of about 20.5 % (w/v)/(w/w), of about 21.0 % (w/v)/(w/w), of about 21.5 % (w/v)/(w/w), of about 22.0 % (w/v)/(w/w), or of about 22.5 % (w/v)/(w/w), or any range formed by any of two of these values.
21. Pharmaceutical composition for use according to any of items 1-20, further comprising one or more cyclodextrin(s), selected from α-cyclodextrins, β-cyclodextrins, γ-cyclodextrins, δ-cyclodextrins and ε-cyclodextrins, preferably from β-cyclodextrins, including hydroxypropyl-β-cyclodextrin (HP-β-CD).
22. Pharmaceutical composition for use according to any of items 1-21, comprising the cyclodextrin(s) in an amount of about 0.1 % (w/v) to about 30 % (w/v), typically in an amount of about 1 % (w/v) to about 20 % (w/v), preferably in an amount of about 2 % (w/v) to about 20 % (w/v), more preferably in an amount of about 5 % (w/v) to about 20 % (w/v), even more preferably in an amount of about 5 % (w/v) to about 15 % (w/v), and most preferably in an amount of about 5 % (w/v) to about 10 % (w/v) or in an amount of about 2 % (w/v) to about 6 % (w/v).
23. Pharmaceutical composition for use according to any one of items 1-22, comprising acetic acid and/or lactic acid in a concentration of about 0.025 to about 0.2 M, preferably in a concentration of about 0.05 to about 0.150 M, imiquimod in an amount of about 0.1 % (w/v) to about 1 % (w/v), cyclodextrin(s) in an amount of about 2 % (w/v) to about 8 % (w/v) and Poloxamer 407 in an amount of about 10 % (w/v) to about 25 % (w/v), preferably in an amount of about 12 % (w/v) to about 25 % (w/v).
24. Pharmaceutical composition for use according to any one of items 1-23, wherein the composition comprises imiquimod in a concentration of about 0.1% - 0.8% (w/w), preferably 0.2 % (w/v) to about 0.5 % (w/v), more preferably 0.2 % - 0.4 % (w/v), Poloxamer 407 in an amout of about 12%-18% (w/w), hydroxypropyl-p-cyclodextrin in an amount of about 2% - 8% (w/v) and lactic acic in an amount of about 0.27% - 0.90 % (w/v).
25. Pharmaceutical composition for use according to any one of items 1-24, wherein the composition comprises imiquidmod in an amount of about 0.4 % (w/v), Poloxamer 407 in an amount of about 16 % (w/v), hydroxypropyl-β-cyclodextrin in an amount of about 5% (w/v) and lactic acid in an amount of about 0.51 % (w/v).
26. Pharmaceutical composition for use according to any one of items 1-25, wherein the composition is an aqueous solution and wherein the pH of the solution is in a range of about pH 4.0 - 5.0, preferably in a range of about pH 4.1 - 4.7.
27. Use of a pharmaceutical composition according to any one of items 1-26 for intravesical administration in the treatment and/or in aiding in the treatment of carcinoma in situ bladder cancer.
28. Use of a pharmaceutical composition according to item 27, wherein the pharmaceutical composition is administered for at least 3 weeks, preferably for at least 4, 5, 6, 7 or for at least 8 weeks, more preferred for about 6-12 weeks, even more preferred for about 6-8 weeks.
29. Use according to any one of items 27- 28, wherein the pharmaceutical composition is administered at time intervals of about 2-7 days, preferably at time intervals of 2, 3, 4, 5, or 6 days, more preferred at time intervals of 3-6 days or at time intervals of 7 days.
30. Use according to any one of items 27-29, wherein the pharmaceutical composition is administered for a total of at least 3, 4, 5, 6, 7 or at least 8 doses, or for 3-12 doses, of 4-11 doses, of 5-10 doses, of 6-9 doses, of 7-8 doses, or of 10, 12, 14, 16, 18, 20, 22, 24, 26, 28, 30, 32, 34 or 36 doses, or of about 3-36 doses, of about 4-32 doses, of about 6-30 doses, of about 8-28 doses, of about 10-26 doses, of about 12-24 doses, of about 14-20 doses, of about 16-22 doses, of about 18-20 doses, or of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or of 36 doses.
31. Use according to any one of items 27-30, wherein the pharmaceutical composition is administered in a volume of about 10-100ml, preferably in a volume of about 20ml, 30ml, 40ml, 50ml, 60ml, 70ml, 80ml, or of about 90ml, or in a volume of about 25ml-100ml, of about 30ml-1 00ml, of about 35ml-1 00ml, of about 40ml-1 00ml, of about 45ml-1 00ml, of about 50ml-1 00ml, of about 55ml-100ml, of about 60ml-1 00ml, of about 65ml-1 00ml, of about 70ml-1 00ml, of about 75ml-100ml, of about 80ml-100ml, of about 85ml-100ml, of about 90ml-100ml, of about 95ml-100ml, or in a volume of e.g. about 25-50ml, of about 30-55ml, of about 35-60ml, of about 40ml-65ml, of about 45ml-70ml, of about 50ml-75ml, of about 55ml-80ml, of about 60ml-85ml, of about 65ml-90ml, of about 70ml-95ml, more preferably in a volume of about 40ml-70ml, or in a volume of about 50ml.
32. Use according to any one of items 27-31, wherein the intravesical retention time is at least about 0.5h, 1h, 1.5h, 2h, 2.5h, 3h, or of at least about 3.5h, or of about 4h, preferably of about 0.5h to about 2h.
33. Use of a pharmaceutical composition according to any one of items 27 - 32, wherein the pharmaceutical composition is administered in a combination therapy, wherein (i) the pharmaceutical composition according to the present invention is administered to treat the CIS bladder cancer and (ii) a concurrent exophytic tumour is treated concomitantly.
34. Method of treatment of carcinoma in situ of the bladder comprising administering to a person in need thereof a therapeutically effective amount of the pharmaceutical composition according to any one of items 1-33.
35. Method according to item 34, wherein the pharmaceutical composition is administered intravesically.
36. Method according to item 34, wherein the pharmaceutical composition is administered at time intervals of about 2-7 days, preferably at time intervals of 2, 3, 4, 5, or 6 days, more preferred at time intervals of 3-6 days or at time intervals of 7 days.
37. Method according to item 36, wherein the pharmaceutical composition is administered intravesically over 1min - 60min, preferably over 2min - 15 min, or over 3min - 15 min, or over 4min - 12min, or over 5min - 1 0min, more preferably over 3 min - 9min, or over 3min - 8min, or 4min - 8 min, most preferably over 3min - 5min.
38. Method according to item 37, wherein the inventive pharmaceutical composition is retained in the bladder for about 0,5 - 2 hours, preferably for about 1 hour.

## Claims

1. Pharmaceutical composition comprising imiquimod and optionally further comprising at least one pharmaceutically acceptable excipient and optionally a vehicle for use in the treatment of carcinoma in situ (CIS) of the bladder.

2. Pharmaceutical composition for use according to claim 1, wherein the carcinoma in situ (CIS) of the bladder is selected from the group consisting of primary carcinoma in situ (CIS) of the bladder, secondary carcinoma in situ (CIS) of the bladder and, preferably, concurrent carcinoma in situ (CIS) of the bladder.

3. Pharmaceutical composition for use according any one of claims 1 or 2 comprising imiquimod in an amount of about 0.005 % (w/v) to about 2 % (w/v), including an amount of about 0.01 % (w/v) to about 2 % (w/v), an amount of about 0.1 % (w/v) to about 1.5 % (w/v), an amount of about 0.1 % (w/v) to about 1 % (w/v), an amount of about 0.2 % (w/v) to about 0.9 % (w/v), an amount of about 0.3 % (w/v) to about 0.9 % (w/v), an amount of about 0.4 % (w/v) to about 0.8 % (w/v), an amount of about 0.5 % (w/v) to about 1 % (w/v) or an amount of about 0.2 % (w/v) to about 1 % (w/v), preferably in an amount of about 0.1 % (w/v) to about 0.5 (w/v).

4. Pharmaceutical composition for use according to any one of claims 1-3 for use in the treatment of a CIS bladder cancer patient in the age of 40 years or older, preferably in the age of 60 years and older, and/or wherein the CIS bladder cancer patient is a male CIS bladder cancer patient.

5. Pharmaceutical composition for use according to any one of claims 1-4, wherein the CIS bladder cancer patients exhibits at least 2 CIS bladder tumour sites in the bladder mucosa.

6. Pharmaceutical composition for use according to any of claims 1-5, further comprising at least one organic acid, wherein the pharmaceutical composition preferably comprises acetic acid and/or lactic acid in a concentration of about 0.025 M to about 0.200 M, preferably in a concentration of about 0.025 M to about 0.100 M, or in a concentration of about 0.035 M to about 0.1 M, or in a concentration of about 0.045 M to about 0.1 M, or in a concentration of about 0.055 M to about 0.1 M, or in a concentration of about 0.065 M to about 0.1 M, or in a concentration of about 0.075 M to about 0.1 M, or in a concentration of about 0.085 M to about 0.1 M, or in a concentration of about 0.100 M to about 0.200 M, or in a concentration of about 0.100 M to 0.150 M, or in a concentration of about 0.075 to about 0.200 M

7. Pharmaceutical composition for use according to any one of claims 1-6 further comprising at least one thermo-sensitive agent, wherein the at least one thermo-sensitive agent preferably exhibits a specific "lower critical solution temperature" (LCST) in a range of about 15°C to about 35°C, more preferably in a range of about 15°C to about 30°C, even more preferably in a range of about 15 or 20°C or 25°C to about 30°C, most preferably in a range of about 15 or 20°C to about 25°C and/or
wherein the pharmaceutical composition preferably comprises the least one thermo-sensitive agent in an amount of about 0.1 % (w/v) to about 40 % (w/v), typically in an amount of about 2 % (w/v) to about 30 % (w/v), preferably in an amount of about 5 % (w/v) to about 30 % (w/v), more preferably in an amount of about 10 % (w/v) to about 30 % (w/v), and most preferably in an amount of about 10 % (w/v) to about 25 % (w/v) or in an amount of about 10 % (w/v) to about 20 % (w/v).

8. Pharmaceutical composition for use according to any of claims 1-7, wherein the at least one thermo-sensitive agent is selected from a poly(ethylene oxide)-poly(propylene oxide)-poly(ethylene oxide) copolymer (also termed PEO-PPO-PEO or poloxamer), preferably selected from a poly(ethylene oxide)-poly(propylene oxide)-poly(ethylene oxide) copolymer (also termed PEO-PPO-PEO or poloxamer) including Pluronic F 108 Cast Solid Surfacta; Pluronic F 108 Pastille; Pluronic F 108 Prill; Pluronic F 108NF Prill (Poloxamer 338); Pluronic F 127; Pluronic F 127 Prill; Pluronic F 127 NF; Pluronic F 127 NF 500 BHT Prill; Pluronic F 127 NF Prill (Poloxamer 407); Pluronic F 38; Pluronic F 38 Pastille; Pluronic F 68; Pluronic F 68 Pastille; Pluronic F 68 LF Pastille; Pluronic F 68 NF Prill (Poloxamer 188); Pluronic F 68 Prill; Pluronic F 77; Pluronic F 77 Micropastille; Pluronic F 87; Pluronic F 87 NF Prill (Poloxamer 237); Pluronic F 87 Prill; Pluronic F 88 Pastille; Pluronic F 88 Prill; Pluronic F 98; Pluronic F 98 Prill; Pluronic L 10; Pluronic L 101; Pluronic L 121; Pluronic L 31; Pluronic L 35; Pluronic L 43; Pluronic L 44; Pluronic L 44 NF (Poloxamer 124); Pluronic L 61; Pluronic L 62; Pluronic L 62 LF; Pluronic L 62D; Pluronic L 64; Pluronic L 81; Pluronic L 92; Pluronic L44 NF INH surfactant (Poloxamer 124); Pluronic N 3; Pluronic P 103; Pluronic P 104; Pluronic P 105; Pluronic P 123 Surfactant; Pluronic P 65; Pluronic P 84; Pluronic P 85; and Poloxamer 403, or is selected from a mixture formed by any two or more of the afore defined thermo-sensitive agents, more preferably the at least one thermo-sensitive agent is selected from Poloxamer 407 and/or Poloxamer 188 or a mixture of Poloxamer 407 and Poloxamer 188, or
is selected from chitosan or its derivatives.

9. Pharmaceutical composition for use according to any one of claims 1-8, wherein the at least one thermo-sensitive agent is Poloxamer 407 in an amount of about 10 % (w/v) to about 25 % (w/v) or in an amount of about 12 % (w/v) to about 25 % (w/v), or in an amount of 14 % (w/v) to about 25 % (w/v), or in an amount of 15 % (w/v) to about 20% (w/v).

10. Pharmaceutical composition for use according to any one of claims 1-9, further comprising one or more cyclodextrin(s), selected from α-cyclodextrins, β-cyclodextrins, γ-cyclodextrins, δ-cyclodextrins and ε-cyclodextrins, preferably from β-cyclodextrins, including hydroxypropyl-β-cyclodextrin (HP-β-CD), preferably comprising the cyclodextrin(s) in an amount of about 0.1 % (w/v) to about 30 % (w/v), typically in an amount of about 1 % (w/v) to about 20 % (w/v), preferably in an amount of about 2 % (w/v) to about 20 % (w/v), more preferably in an amount of about 5 % (w/v) to about 20 % (w/v), even more preferably in an amount of about 5 % (w/v) to about 15 % (w/v), and most preferably in an amount of about 5 % (w/v) to about 10 % (w/v) or in an amount of about 2 % (w/v) to about 6 % (w/v).

11. Pharmaceutical composition for use according to any one of claims 1-10, wherein the composition comprises imiquimod in a concentration of about 0.1% - 0.8% (w/w), preferably 0.2 % (w/v) to about 0.5 % (w/v), more preferably 0.2 % - 0.4 % (w/v), Poloxamer 407 in an amout of about 12%-18% (w/w), hydroxypropyl-β-cyclodextrin in an amount of about 2% - 8% (w/v) and lactic acic in an amount of about 0.27% - 0.90 % (w/v).

12. Pharmaceutical composition for use according to any one of claims 1-11, wherein the composition is an aqueous solution and wherein the pH of the solution is in a range of about pH 4.0 - 5.0, preferably in a range of about pH 4.1 - 4.7.

13. Pharmaceutical composition for use according to any one of claims 1-12 for intravesical administration.

14. Pharmaceutical composition for use according to claim 13, wherein the pharmaceutical composition is administered for at least 3 weeks, preferably for at least 4, 5, 6, 7 or for at least 8 weeks, more preferred for about 6-12 weeks, even more preferred for about 6-8 weeks and/or wherein the pharmaceutical composition is administered at time intervals of about 2-7 days, preferably at time intervals of 2, 3, 4, 5, or 6 days, more preferred at time intervals of 3-6 days or at time intervals of 7 days.

15. Pharmaceutical composition for use according to any one of claims 13 or 14, wherein the pharmaceutical composition is administered for a total of at least 3, 4, 5, 6, 7 or at least 8 doses, or for 3-12 doses, of 4-11 doses, of 5-10 doses, of 6-9 doses, of 7-8 doses, or of 10, 12, 14, 16, 18, 20, 22, 24, 26, 28, 30, 32, 34 or 36 doses, or of about 3-36 doses, of about 4-32 doses, of about 6-30 doses, of about 8-28 doses, of about 10-26 doses, of about 12-24 doses, of about 14-20 doses, of about 16-22 doses, of about 18-20 doses, or of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35 or of 36 doses and/or
wherein the pharmaceutical composition is administered in a volume of about 10-100ml, preferably in a volume of about 20ml, 30ml, 40ml, 50ml, 60ml, 70ml, 80ml, or of about 90ml, or in a volume of about 25ml-100ml, of about 30ml-100ml, of about 35ml-100ml, of about 40ml-1 00ml, of about 45ml-1 00ml, of about 50ml-1 00ml, of about 55ml-100ml, of about 60ml-100ml, of about 65ml-100ml, of about 70ml-100ml, of about 75ml-100ml, of about 80ml-100ml, of about 85ml-100ml, of about 90ml-100ml, of about 95ml-100ml, or in a volume of e.g. about 25-50ml, of about 30-55ml, of about 35-60ml, of about 40ml-65ml, of about 45ml-70ml, of about 50ml-75ml, of about 55ml-80ml, of about 60ml-85ml, of about 65ml-90ml, of about 70ml-95ml, more preferably in a volume of about 40ml-70ml, or in a volume of about 50ml and/or wherein the intravesical retention time is at least about 0.5h, 1h, 1.5h, 2h, 2.5h, 3h, or of at least about 3.5h, or of about 4h, preferably of about 0.5h to about 2h.

16. A pharmaceutical composition for use according to any one of claims 13 - 15, wherein the pharmaceutical composition is administered in a combination therapy, wherein (i) the pharmaceutical composition is administered to treat CIS bladder cancer and (ii) a concurrent exophytic tumour is treated concomitantly.
